# EUROPEAN PATENT SPECIFICATION

(11) **EP 2 294 220 B1**
(45) Date of publication and mention of the grant of the patent: **20.04.2016**
(21) Application number: 09761803.7
(22) Date of filing: 15.06.2009
(51) Int. Cl.: C12Q 1/68

(54) **P2/P2A/P2B GENE SEQUENCES AS DIAGNOSTIC TARGETS FOR THE IDENTIFICATION OF FUNGAL AND YEAST SPECIES**
P2/P2A/P2B-GENSEQUENZEN ALS DIAGNOSTISCHE ZIELE FÜR DIE IDENTIFIZIERUNG VON PILZ- UND HEFESPEZIES
SÉQUENCES DE GÈNES P2/P2A/P2B EN TANT QUE CIBLES DE DIAGNOSTIC POUR L'IDENTIFICATION D'ESPÈCES FONGIQUES ET DE LEVURE

(30) Priority: 13.06.2008 IE 20080489
(43) Date of publication of application: 16.03.2011
(73) Proprietor: National University of Ireland, Galway, Galway (IE)
(72) Inventor: BARRY, Thomas, Gerard, Co. Galway (IE); SMITH, Terry, James, Galway (IE); JANKIEWICZ, Marcin, Co. Clare (IE); O'CONNOR, Louise, Co. Galway (IE); TUITE, Nina, Galway (IE); LAHIFF, Sinead, Galway (IE); MAHER, Majella, Co. Galway (IE)
(74) Representative: Gates, Marie Christina Esther
(86) International application number: PCT/EP2009/057338
(87) International publication number: WO 2009/150242

(56) References cited:
- US-B1- 6 747 137
- NAILIS HELEEN ET AL: "Development and evaluation of different normalization strategies for gene expression studies in Candida albicans biofilms by real-time PCR" BMC MOLECULAR BIOLOGY, BIOMED CENTRAL LTD, GB, vol. 7, no. 1, 4 August 2006 (2006-08-04), page 25, XP021014974 ISSN: 1471-2199
- DATABASE EMBL [Online] 20 November 2000 (2000-11-20), "Candida albicans 60S acidic ribosomal protein type P2-B (p2B) gene, complete cds." XP002556024 retrieved from EBI accession no. EMBL:AF317662 Database accession no. AF317662 & ABRAMCZYK D ET AL: "Overexpression, purification and characterization of the acidic ribosomal P-proteins from Candida albicans" BIOCHIMICA ET BIOPHYSICA ACTA - GENERAL SUBJECTS, ELSEVIER SCIENCE PUBLISHERS, NL, vol. 1672, no. 3, 11 June 2004 (2004-06-11), pages 214-223, XP004514841 ISSN: 0304-4165
- DATABASE EMBL [Online] 20 November 2000 (2000-11-20), "Candida albicans 60S acidic ribosomal protein type P2-A (p2A) gene, complete cds." XP002556025 retrieved from EBI accession no. EMBL:AF317661 Database accession no. AF317661 & ABRAMCZYK D ET AL: "Overexpression, purification and characterization of the acidic ribosomal P-proteins from Candida albicans" BIOCHIMICA ET BIOPHYSICA ACTA - GENERAL SUBJECTS, ELSEVIER SCIENCE PUBLISHERS, NL, vol. 1672, no. 3, 11 June 2004 (2004-06-11), pages 214-223, XP004514841 ISSN: 0304-4165
- ABRAMCZYK D ET AL: "Overexpression, purification and characterization of the acidic ribosomal P-proteins from Candida albicans" BIOCHIMICA ET BIOPHYSICA ACTA - GENERAL SUBJECTS, ELSEVIER SCIENCE PUBLISHERS, NL, vol. 1672, no. 3, 11 June 2004 (2004-06-11), pages 214-223, XP004514841 ISSN: 0304-4165 cited in the application
- DATABASE Geneseq [Online] 19 October 1998 (1998-10-19), "A. fumigatus allergen rAsp f8 encoding cDNA." XP002556026 retrieved from EBI accession no. GSN:AAV45117 Database accession no. AAV45117 & WO 98/28624 A (PHARMACIA & UPJOHN DIAG AB [SE]; CRAMERI RETO [CH]; HEMMANN STEPANIE [) 2 July 1998 (1998-07-02)
- SCHWIENBACHER M ET AL: "Asp f6, an Aspergillus allergen specifically recognized by IgE from patients with allergic bronchopulmonary aspergillosis, is differentially expressed during germination." ALLERGY NOV 2005, vol. 60, no. 11, November 2005 (2005-11), pages 1430-1435, XP002556021 ISSN: 0105-4538
- MAYER C ET AL: "Humoral and cell-mediated autoimmune reactions to human acidic ribosomal P2 protein in individuals sensitized to Aspergillus fumigatus P2 protein." THE JOURNAL OF EXPERIMENTAL MEDICINE 3 MAY 1999, vol. 189, no. 9, 3 May 1999 (1999-05-03), pages 1507-1512, XP002556022 ISSN: 0022-1007
- ABRAMCZYK DARIUSZ ET AL: "Non-AUG translation initiation of mRNA encoding acidic ribosomal P2A protein in Candida albicans." YEAST (CHICHESTER, ENGLAND) SEP 2003, vol. 20, no. 12, September 2003 (2003-09), pages 1045-1052, XP002556023 ISSN: 0749-503X
- DATABASE EMBL [Online] 10 October 2004 (2004-10-10), "Sequence 5594 from patent US 6747137." XP002556027 retrieved from EBI accession no. EMBL:AR550463 Database accession no. AR550463 & US 6 747 137 B1 (WEINSTOCK KEITH G [US] ET AL) 8 June 2004 (2004-06-08)

## Description

### Field of the Invention

The present invention relates to nucleic acid primers and probes to detect one or more fungal and yeast species. More specifically the invention relates to the P2, P2A and P2B gene sequences (also known as 60S acidic ribosomal protein P2, RLA-2-ASPFU, Allergen ASP f8 or Afp2), the corresponding RNA, specific probes, primers and oligonucleotides related thereto and their use in diagnostic assays to detect and/ or discriminate fungal and yeast species, as defined in the appended claims.

### Background to the Invention

Yeast and fungal infections represent a major cause of morbidity and mortality among immunocompromised patients. The number of immunocompromised patients at risk of yeast and fungal infection continues to increase each year, as does the spectrum of fungal and yeast agents causing disease. Mortality from fungal infections, particularly invasive fungal infections, is 30% or greater in certain risk groups. The array of available anti-fungal agents is growing; however, so too is the recognition of both intrinsic and emerging resistance to antifungal drugs. These factors are contributing to the increased need for cost containment in laboratory testing and have led to laboratory consolidation in testing procedures.

Invasive fungal infections are on the increase. In 2003, it was estimated that there were 9 million at risk patients of which 1.2 million developed infection. *Candida* spp. and *Aspergillus* spp. now rank as the most prominent pathogens infecting immunosupressed patients. In particular, infections are common in the urinary tract, the respiratory system and the bloodstream, at the site of insertion of stents, catheters and orthopaedic joints. Approximately, 10% of the known *Candida* spp. have been implicated in human infection. Invasive candidiasis occurs when candida enters the bloodstream and it is estimated to occur at a frequency of 8/100,000 population in the US with a mortality rate of 40%. *Candida albicans* is the 4^{th} most common cause of bloodstream infection. Aspergillosis usually begins as a pulmonary infection that can progress to a life-threatening invasive infection in some patients and has a mortality rate of greater than 90%. Emerging mycoses agents include *Fusarium, Scedosporium, Zygomycetes* and *Trichosporon* spp. *("*Stakeholder Insight: Invasive fungal infections", Datamonitor, Jan 2004).

Immunocompromised patients including transplant and surgical patients, neonates, cancer patients, diabetics and those with HIV/AIDs are at high risk of developing invasive fungal infections (Datamonitor report: Stakeholder opinion -Invasive fungal infections, options outweigh replacements 2004). A large number of severe cases of sepsis are reported each year. Despite improvements in its medical management, sepsis still constitutes one of the greatest challenges in intensive care medicine. Microorganisms (bacteria, fungi and yeast) responsible for causing sepsis are traditionally detected in hospital laboratories with the aid of microbiological culture methods with poor sensitivity (25-82%), which are very time-consuming, generally taking from two to five days to complete, and up to eight days for the diagnosis of fungal infections. Definitive diagnosis of an infection caused by a yeast or fungus is usually based on either, the recovery and identification of a specific agent from clinical specimens or microscopic demonstration of fungi with distinct morphological features. However, there are numerous cases where these methods fail to provide conclusive proof as to the infecting agent. In these instances, the detection of specific host antibody responses can be used, although again this can be affected by the immune status of the patient. Time is critical in the detection and identification of bloodstream infections typically caused by bacteria and fungi. Effective treatment depends on finding the source of infection and making appropriate decisions about antibiotics or antifungals quickly and efficiently. Only after pathogens are correctly identified can targeted therapy using a specific antibiotic or anti-fungal begin. Many physicians would like to see the development of better in vitro amplification and direct detection diagnostic techniques for the early diagnosis of yeast and fungi (*"*Stakeholder Insight: Invasive fungal infections", Datamonitor, Jan 2004). Recently Roche™ launched a real time PCR based assay (Septifast^{tm}), for the detection of bacterial, fungal and yeast DNA in clinical samples. Therefore, there is a clear need for the development of novel rapid diagnostic tests for clinically significant bacterial and fungal pathogens for bioanalysis applications in the clinical sector. This has led the current inventors to identify novel fungal and yeast nucleic acid targets for application in Nucleic Acid Diagnostics (NAD) tests. Fungal and yeast nucleic acid based diagnostics have focused heavily on the ribosomal RNA (rRNA) genes, RNA transcripts, and their associated DNA / RNA regions. The rRNA genes are highly conserved in all fungal species and they also contain divergent and distinctive intergenic transcribed spacer regions. Ribosomal rRNA comprises three genes: the large sub-unit gene (28S), the small sub-unit gene (18S) and the 5.8S gene. The 28S and 18S rRNA genes are separated by the 5.8S rRNA and two internal transcribed spacers (ITS1 and ITS2). Because the ITS region contains a high number of sequence polymorphisms, numerous researchers have concentrated their efforts on these as targets (Atkins and Clark, 2004). rRNA genes are also multicopy genes with >10 copies within the fungal genome.

A number of groups are working on developing new assays for fungal and yeast infections. US2004044193 relates to, amongst a number of other aspects, the transcription factor CaTEC1 of *Candida albicans;* inhibitors thereof, and methods for the diagnosis and therapy of diseases which are connected with a Candida infection; and also diagnostic and pharmaceutical compositions which contain the nucleotide sequences, proteins, host cells and/or antibodies. WO0183824 relates to hybridization assay probes and accessory oligonucleotides for detecting ribosomal nucleic acids from *Candida albicans* and/or *Candida dubliniensis.* US6017699 and US5426026 relate to a set of DNA primers, which can be used to amplify and speciate DNA from five medically important Candida species. US 6747137 discloses sequences useful for diagnosis of Candida infections. EP 0422872 and US 5658726 disclose probes based on 18S rRNA genes, and US 5958693 discloses probes based on 28S rRNA, for diagnosis of a range of yeast and fungal species. US 6017366 describes sequences based on chitin synthase gene for use in nucleic acid based diagnostics for a range of Candida species. Nailis et al. (BMC Molecular Biology 2006,7:25) discloses primers and a probe for real time PCR to detect the C. albicans RPP2B gene. It is clear though, that development of faster, more accurate diagnostic methods are required, particularly in light of the selection pressure caused by modem anti-microbial treatments which give rise to increased populations of resistant virulent strains with mutated genome sequences. Methods that enable early diagnosis of microbial causes of infection enable the selection of a specific narrow spectrum antibiotic or antifungal to treat the infection (Datamonitor report: Stakeholder opinion -Invasive fungal infections, options outweigh replacements 2004; Datamonitor report: Stakeholder Opinion-Sepsis, under reaction to an overreaction, 2006).

Ribosomes are a two part organelle, composed of a large and a small subunit. The large subunit possesses a number of features which are conserved in all organisms for example the presence of a complex of a number of acidic proteins, the P proteins which form a stalk-like structure (Abramczyk *et al.,* 2004 Tchórzewski *et al.,* 2000; 2003). In the 60S ribosomal subunit of eukaryotes these acidic P proteins include, P0, P1 and P2 (Wool et al 1991). The complex formed by these P proteins is required for efficient translation. In yeasts, there are two isoforms of P1 (P1A and P1B) and P2 (P2A and P2B) which are encoded by four distinct single copy genes (Newton et al, 1990, Bailey-Serres *et al.,* 1997).

This disclosure relates to the use of the P2A, P2B genes in yeast and the P2 gene in fungi. There are currently 65 P2B gene sequences (approx. 360 bp) available in NCBI GenBank database including 3 P2B sequences for *Candida albicans* and one sequence of high homology for *C*. *glabrata.* There are 329 P2 sequences available in NCBI GenBank database including annotated sequences for two *Aspergillus* species and presumptive P2 sequences for three species. Additional P2B sequences of representative species of *Candida* and P2 sequences of representative species of *Aspergillus* were generated by the inventors following the design of PCR primers to amplify P2B gene regions in *Candida* and P2 gene regions in *Aspergillus* spp. The published and newly generated P2B and P2 gene sequences for *Candida* and *Aspergillus* spp. respectively, were aligned and analysed using bioinformatics tools. PCR primers and species-specific DNA probes for selected species, *Candida glabrata* and *Aspergillus fumigatus* were designed and demonstrated for molecular species identification using real-time PCR. These examples demonstrate the potential of P2A/P2B and P2 genes for molecular identification of species of *Candida, Aspergillus* and other yeast and fungal species.

### Definitions

"Synthetic oligonucleotide" refers to molecules of nucleic acid polymers of 2 or more nucleotide bases that are not derived directly from genomic DNA or live organisms. The term synthetic oligonucleotide is intended to encompass DNA, RNA, and DNA/RNA hybrid molecules that have been manufactured chemically, or synthesized enzymatically *in vitro.*

An "oligonucleotide" is a nucleotide polymer having two or more nucleotide subunits covalently joined together. Oligonucleotides are generally about 10 to about 100 nucleotides. The sugar groups of the nucleotide subunits may be ribose, deoxyribose, or modified derivatives thereof such as OMe. The nucleotide subunits may be joined by linkages such as phosphodiester linkages, modified linkages or by non-nucleotide moieties that do not prevent hybridization of the oligonucleotide to its complementary target nucleotide sequence. Modified linkages include those in which a standard phosphodiester linkage is replaced with a different linkage, such as a phosphorothioate linkage, a methylphosphonate linkage, or a neutral peptide linkage. Nitrogenous base analogs also may be components of oligonucleotides in accordance with the invention. A "target nucleic acid" is a nucleic acid comprising a target nucleic acid sequence. A "target nucleic acid sequence," "target nucleotide sequence" or "target sequence" is a specific deoxyribonucleotide or ribonucleotide sequence that can be hybridized to a complementary oligonucleotide.

An "oligonucleotide probe" is an oligonucleotide having a nucleotide sequence sufficiently complementary to its target nucleic acid sequence to be able to form a detectable hybrid probe:target duplex under high stringency hybridization conditions. An oligonucleotide probe is an isolated chemical species and may include additional nucleotides outside of the targeted region as long as such nucleotides do not prevent hybridization under high stringency hybridization conditions. Non-complementary sequences, such as promoter sequences, restriction endonuclease recognition sites, or sequences that confer a desired secondary or tertiary structure such as a catalytic active site can be used to facilitate detection using the invented probes. An oligonucleotide probe optionally may be labelled with a detectable moiety such as a radioisotope, a fluorescent moiety, a chemiluminescent, a nanoparticle moiety, an enzyme or a ligand, which can be used to detect or confirm probe hybridization to its target sequence. Oligonucleotide probes are preferred to be in the size range of from about 10 to about 100 nucleotides in length, although it is possible for probes to be as much as and above about 500 nucleotides in length, or below 10 nucleotides in length.

A "hybrid" or a "duplex" is a complex formed between two single-stranded nucleic acid sequences by Watson-Crick base pairings or non-canonical base pairings between the complementary bases. "Hybridization" is the process by which two complementary strands of nucleic acid combine to form a double-stranded structure ("hybrid" or "duplex"). A "fungus" or "yeast" is meant any organism of the kingdom Fungi, and preferably, is directed towards any organism of the phylum Ascomycota. "Complementarity" is a property conferred by the base sequence of a single strand of DNA or RNA which may form a hybrid or double-stranded DNA:DNA, RNA:RNA or DNA:RNA through hydrogen bonding between Watson-Crick base pairs on the respective strands. Adenine (A) ordinarily complements thymine (T) or uracil (U), while guanine (G) ordinarily complements cytosine (C).

The term "stringency" is used to describe the temperature, ionic strength and solvent composition existing during hybridization and the subsequent processing steps. Those skilled in the art will recognize that "stringency" conditions may be altered by varying those parameters either individually or together. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. Stringency conditions are chosen to maximize the difference in stability between the hybrid formed with the target and the non-target nucleic acid. With "high stringency" conditions, nucleic acid base pairing will occur only between nucleic acid fragments that have a high frequency of complementary base sequences (for example, hybridization under "high stringency" conditions, may occur between homologs with about 85-100% identity, preferably about 70-100% identity). With medium stringency conditions, nucleic acid base pairing will occur between nucleic acids with an intermediate frequency of complementary base sequences (for example, hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

'High stringency' conditions are those equivalent to binding or hybridization at 42° C. in a solution consisting of 5xSSPE (43.8g/l NaCl, 6.9 g/l NaH₂PO₄H₂O and 1.85 g/l EDTA, ph adjusted to 7.4 with NaOH), 0.5% SDS, 5xDenhardt's reagent and 100µg/ml denatured salmon sperm DNA followed by washing in a solution comprising 0.1xSSPE, 1.0%SDS at 42° C. when a probe of about 500 nucleotides in length is used. "Medium stringency' conditions are those equivalent to binding or hybridization at 42° C. in a solution consisting of 5XSSPE (43.8 g/l NaCl, 6.9 g/l NaH₂PO₄H₂O and 1.85 g/l EDTA, pH adjusted to 7.4 with NaOH), 0.5% SDS, 5xDenhardt's reagent and 100 µg/ml denatured salmon sperm DNA followed by washing in a solution comprising 1.0xSSPE, 1.0% SDS at 42° C, when a probe of about 500 nucleotides in length is used. 'Low stringency' conditions are those equivalent to binding or hybridization at 42° C. in a solution consisting of 5xSSPE (43.8 g/l NaCl, 6.9 g/l NaH₂PO₄H₂O and 1.85 g/l EDTA, pH adjusted to 7.4 with NaOH), 0.1% SDS, 5xDenhardt's reagent [50xDenhardt's contains per 500ml: 5g Ficoll (Type 400, Pharamcia), 5 g BSA (Fraction V; Sigma)] and 100 µg/ml denatured salmon sperm DNA followed by washing in a solution comprising 5xSSPE, 0.1% SDS at 42° C, when a probe of about 500 nucleotides in length is used.

In the context of nucleic acid *in-vitro* amplification based technologies, "stringency" is achieved by applying temperature conditions and ionic buffer conditions that are particular to that *in-vitro* amplification technology. For example, in the context of PCR and real-time PCR, "stringency" is achieved by applying specific temperatures and ionic buffer strength for hybridisation of the oligonucleotide primers and, with regards to real-time PCR hybridisation of the probe/s, to the target nucleic acid for *in-vitro* amplification of the target nucleic acid.

One skilled in the art will understand that substantially corresponding probes of the invention can vary from the referred-to sequence and still hybridize to the same target nucleic acid sequence. This variation from the nucleic acid may be stated in terms of a percentage of identical bases within the sequence or the percentage of perfectly complementary bases between the probe and its target sequence. Probes of the present invention substantially correspond to a nucleic acid sequence if these percentages are from about 100% to about 80% or from 0 base mismatches in about 10 nucleotide target sequence to about 2 bases mismatched in an about 10 nucleotide target sequence. In preferred embodiments, the percentage is from about 100% to about 85%. In more preferred embodiments, this percentage is from about 90% to about 100%; in other preferred embodiments, this percentage is from about 95% to about 100%

By "sufficiently complementary" or "substantially complementary" is meant nucleic acids having a sufficient amount of contiguous complementary nucleotides to form, under high stringency hybridization conditions, a hybrid that is stable for detection.

The terms "identical" or percent "identity," in the context of two or more nucleic acids or polypeptide sequences, refer to two or more sequences or subsequences that are the same or have a specified percentage of amino acid residues or nucleotides that are the same (*i.e*., 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.,* NCBI web site at ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1987-2005, Wiley Interscience)). A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra).* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

By "nucleic acid hybrid" or "probe:target duplex" is meant a structure that is a double-stranded, hydrogen-bonded structure, preferably about 10 to about 100 nucleotides in length, more preferably 14 to 50 nucleotides in length, although this will depend to an extent on the overall length of the oligonucleotide probe. The structure is sufficiently stable to be detected by means such as chemiluminescent or fluorescent light detection, autoradiography, electrochemical analysis or gel electrophoresis. Such hybrids include RNA:RNA, RNA:DNA, or DNA:DNA duplex molecules.

"RNA and DNA equivalents" refer to RNA and DNA molecules having the same complementary base pair hybridization properties. RNA and DNA equivalents have different sugar groups (i.e., ribose versus deoxyribose), and may differ by the presence of uracil in RNA and thymine in DNA. The difference between RNA and DNA equivalents do not contribute to differences in substantially corresponding nucleic acid sequences because the equivalents have the same degree of complementarity to a particular sequence.

By "preferentially hybridize" is meant that under high stringency hybridization conditions oligonucleotide probes can hybridize their target nucleic acids to form stable probe:target hybrids (thereby indicating the presence of the target nucleic acids) without forming stable probe:non-target hybrids (that would indicate the presence of non-target nucleic acids from other organisms). Thus, the probe hybridizes to target nucleic acid to a sufficiently greater extent than to non-target nucleic acid to enable one skilled in the art to accurately detect the presence of (for example Candida) and distinguish these species from other organisms. Preferential hybridization can be measured using techniques known in the art and described herein.

By "theranostics" is meant the use of diagnostic testing to diagnose the disease, choose the correct treatment regime and monitor the patient response to therapy. The theranostics of the invention may be based on the use of an NAD assay of this invention on samples, swabs or specimens collected from the patient.

### Object of the Invention

It is an object of the current invention to provide sequences and/or diagnostic assays to detect and identify one or more yeast and fungal species. The current inventors have made use of the P2, P2A and P2B gene sequences to design primers that are specific to *Candida* P2B genes and to *Aspergillus* P2 genes. Such primers not only allow the detection of yeast and fungal species but also allow distinction between *Candida* and *Aspergillus* spp. This has an advantage over the prior art in that if one wants to identify a fungal pathogen in a sample, which contains Candida as a commensal, the approach of using universal primers may not be successful. There is a strong possibility that the Candida will out-compete the fungal pathogen in the amplification process and will be preferentially amplified, resulting in failure to detect the disease-causing pathogen. The current invention further provides for primers and probes that allow discrimination between different Candida species and among different Aspergillus species.

### Summary of the Invention

The diagnosic Kits, nucleic acid molecules and methods of the present invention are defined in the appended claims. The present disclosure provides for a diagnostic kit for detection and identification of yeast and/or fungal species, comprising an oligonucleotide probe capable of binding to at least a portion of the P2, P2A or P2B gene or its corresponding mRNA. The oligonucleotide probe may have a sequence substantially homologous to or substantially complementary to a portion of the P2, P2A or P2B gene or its corresponding mRNA. It will thus be capable of binding or hybridizing with a complementary DNA or RNA molecule. The P2 gene may be a fungal P2 gene. The P2A gene may be a yeast gene. The P2B gene may be yeast P2B gene. The nucleic acid molecule may be synthetic. The oligonucleotide probe may have a sequence of SEQ ID NO 9, 10, 91, - 94, 105 - 109, or a sequence substantially homologous to or substantially complementary to those sequences, which can also act as a probe for the P2, P2A or P2B genes.

The kit may comprise more than one such probe. In particular the kit may comprise a plurality of such probes. In addition, the kit may comprise additional probes for other organisms, such as, for example, bacterial species or viruses.

The identified sequences are suitable not only for *in vitro* DNA/RNA amplification based detection systems but also for signal amplification based detection systems. Furthermore, the sequences of the disclosure identified as suitable targets provide the advantages of having significant intragenic sequence heterogeneity in some regions, which is advantageous and enables aspects of the disclosure to be directed towards group or species-specific targets, and also having significant sequence homogeneity in some regions, which enables aspects of the disclosure to be directed towards genus-specific yeast and fungal primers and probes for use in direct nucleic acid detection technologies, signal amplification nucleic acid detection technologies, and nucleic acid in vitro amplification technologies for yeast and fungal diagnostics. The P2, P2A and P2B sequences allow for multi-test capability and automation in diagnostic assays. One of the advantages of the sequences of the present disclosure is that the intragenic P2, P2A and P2B nucleotide sequences diversity between closely related yeast or fungal species enables specific primers and probes for use in diagnostics assays for the detection of yeast and fungi to be designed. The P2, P2A and P2B nucleotide sequences, both DNA and RNA can be used with direct detection, signal amplification detection and in *vitro* amplification technologies in diagnostics assays. The P2, P2A and P2B sequences allow for multi-test capability and automation in diagnostic assays.

The kit may further comprise a primer for amplification of at least a portion of the P2 and/or P2A and/or P2B genes. Suitably, the kit comprises a forward and a reverse primer for a portion of the P2, P2A and/or P2B gene.

The portion of the P2A gene may be equivalent to a portion of the region of the gene from bp position 114 to bp position 439 of *C*. *albicans* (AF317661.1).

The portion of the P2B gene may be equivalent to a portion of the region of the gene from base pair position 1 to position 335 in *C*. *albicans* (XM_718047.1, XM_717900.1) or position 8 to base pair position 359 of the P2B gene in *C*. *albicans* (AF317662.1). *C*. *albicans.*

Particularly preferred, are kits comprising, a probe for a portion of the P2A *C*. *albicans* gene and / or a probe for a portion of the region of the gene equivalent to base pair position 114 to bp position 439 of *C*. *albicans* (AF317661.1).

Particularly preferred, are kits comprising, a probe for a portion of the P2B *C*. *albicans* gene and / or a probe for a portion of the region of the gene equivalent to base pair position 1 to position 359. Also preferred are probes for a portion of the region of the gene equivalent to base pair position 1 to position 335 in *C*. *albicans* (XM_718047.1, MM_717900.1) or position 8 to bp position 359 of the P2B gene in *C. albicans* (AF317662.1). Equivalent base pair positioning may be found in other organisms, but not necessarily in the same position.

The portion of the P2B gene may be equivalent to a portion of the region of the gene from base pair 24 to bp position 158 in *C*. *glabrata* (XM_444905.1). Particularly preferred, are kits comprising, a probe for a portion of the P2B *C*. *glabrata* gene and / or a probe for a portion of the region of the gene equivalent to base pair position 24 to base pair position 158 in *C*. *glabrata.* Sequences equivalent to base pair position 24 to base pair position 158 can be found in other organisms, but not necessarily in the same position. The kit may also comprise additional primers or probes.

The portion of the P2 gene may be equivalent to a portion of the region of the gene from base pair positions 1 to 326 in *A. fumigatus.* Particularly preferred, are kits comprising, a probe for a portion of the P2 *A. fumigatus* gene and / or a probe for a portion of the region of the gene equivalent to base pair position 1 to base pair position 326 in *A. fumigatus.* Equivalent sequences to base pair position 1 to base pair position 326 can be found in other organisms, but not necessarily in the same position. The kit may also comprise additional primers or probes.

The primer may have a sequence selected from the group SEQ ID NO 1 - 8, 85 - 90, 95 - 104, and a sequence substantially homologous to or substantially complementary to those sequences, which can also act as a primer for the P2, P2A and P2B genes. The primers may also be a primer which preferentially hybridizes to the same nucleotide sequence as is preferentially hybridized by the primers SEQ ID NO 1 - 8, 85 - 90, 95-104.

The kit may comprise at least one forward *in vitro* amplification primer and at least one reverse *in vitro* amplification primer, the forward amplification primer having a sequence selected from the group consisting of SEQ ID NO 1, 3, 7, 85 - 88, 95 - 99, and a sequence being substantially homologous or complementary thereto which can also act as a forward amplification primer for the P2A, P2B or P2 genes, and the reverse amplification primer having a sequence selected from the group consisting of SEQ ID NO 2, 4, 5, 6, 8, 89, 90, 100 - 104, and a sequence being substantially homologous or complementary thereto which can also act as a reverse amplification primer for the P2A, P2B or P2 genes

The diagnostic kit may be based on direct nucleic acid detection technologies, signal amplification nucleic acid detection technologies, and nucleic acid *in vitro* amplification technologies is selected from one or more of Polymerase Chain Reaction (PCR), Ligase Chain Reaction (LCR), Nucleic Acids Sequence Based Amplification (NASBA), Strand Displacement Amplification (SDA), Transcription Mediated Amplification (TMA), Branched DNA technology (bDNA) and Rolling Circle Amplification Technology (RCAT)), or other *in vitro* enzymatic amplification technologies.

The disclosure also provides a nucleic acid molecule selected from the group consisting of SEQ ID NO.1 to SEQ ID NO. 110 and sequences substantially homologous thereto, or substantially complementary to a portion thereof and having a function in diagnostics based on the P2 and/or P2A and P2B genes. The nucleic acid molecule may comprise an oligonucleotide having a sequence substantially homologous to or substantially complementary to a portion of a nucleic acid molecule of SEQ ID NO.1 to SEQ ID NO. 110. The disclosure also provides a method of detecting a target organism in a test sample comprising the steps of:
(i) Mixing the test sample with at least one oligonucleotide probe as defined above under appropriate conditions; and
(ii) hybridizing under high stringency conditions any nucleic acid that may be present in the test sample with the oligonucleotide to form a probe:target duplex; and
(iii) determining whether a probe:target duplex is present; the presence of the duplex positively identifying the presence of the target organism in the test sample.

The nucleic acid molecule and kits of the present disclosure may be used in a diagnostic assay to detect the presence of one or more yeast and/or fungal species, to measure yeast and/or fungal titres in a patient or in a method of assessing the efficacy of a treatment regime designed to reduce yeast and/or fungal titre in a patient or to measure yeast and/or fungal contamination in an environment. The environment may be a hospital, or it may be a food sample, an environmental sample e.g. water, an industrial sample such as an in-process sample or an end product requiring bioburden or quality assessment. The kits and the nucleic acid molecule of the disclosure may be used in the identification and/or characterization of one or more disruptive agents that can be used to disrupt the P2A, P2B or P2 gene function. The disruptive agent may be selected from the group consisting of antisense RNA, PNA, and siRNA.

In some embodiments of the disclosure; a nucleic acid molecule comprising a species-specific probe can be used to discriminate between species of the same genus.

The oligonucleotides of the disclosure may be provided in a composition for detecting the nucleic acids of yeast and fungal target organisms. Such a composition may also comprise buffers, enzymes, detergents, salts and so on, as appropriate to the intended use of the compositions. It is also envisioned that the compositions, kits and methods of the invention, while described herein as comprising at least one synthetic oligonucleotide, may also comprise natural oligonucleotides with substantially the same sequences as the synthetic nucleotide fragments in place of, or alongside synthetic oligonucleotides.

The disclosure also provides for an *in vitro* amplification diagnostic kit for a target yeast and/or fungal organism comprising at least one forward *in vitro* amplification primer and at least one reverse *in vitro* amplification primer, the forward amplification primer being selected from the group consisting of one or more of a sequence being substantially homologous or complementary thereto which can also act as a forward amplification primer, and the reverse amplification primer being selected from the group consisting of one or more of or a sequence being substantially homologous or complementary thereto which can also act as a reverse amplification primer.

The disclosure also provides for a diagnostic kit for detecting the presence of candidate yeast and/or fungal species, comprising one or more DNA probes comprising a sequence substantially complementary to, or substantially homologous to the sequence of the P2A, P2B or P2 gene of the candidate yeast and/or fungal species. The present disclosure also provides for one or more synthetic oligonucleotides having a nucleotide sequence substantially homologous to or substantially complementary to one or more of the group consisting of the P2A, P2B gene or mRNA transcripts thereof, the yeast and or fungal P2 gene or mRNA transcript thereof, the yeast P2B gene or mRNA transcript thereof, one or more of SEQ ID NO 1-SEQ ID NO 110.

The nucleotide may comprise DNA. The nucleotide may comprise RNA. The nucleotide may comprise a mixture of DNA, RNA and PNA. The nucleotide may comprise synthetic nucleotides. The sequences of the disclosure (and the sequences relating to the methods, kits compositions and assays of the invention) may be selected to be substantially homologous to a portion of the coding region of the P2, P2A or P2B genes. The gene may be a gene from a target yeast or fungal organism. The sequences of the disclosure are preferably sufficient so as to be able form a probe:target duplex to the portion of the sequence.

The disclosure also provides for a diagnostic kit for a target yeast or fungal organism comprising an oligonucleotide probe substantially homologous to or substantially complementary to an oligonucleotide of the disclosure (which may be synthetic). It will be appreciated that sequences suitable for use as *in vitro* amplification primers may also be suitable for use as oligonucleotide probes: while it is preferable that amplification primers may have a complementary portion of between about 15 nucleotides and about 30 nucleotides (more preferably about 15-about 23, most preferably about 20 to about 23), oligonucleotide probes of the disclosure may be any suitable length. The skilled person will appreciate that different hybridization and or annealing conditions will be required depending on the length, nature & structure (eg. Hybridization probe pairs for LightCycler, Taqman 5' exonuclease probes, hairpin loop structures etc. and sequence of the oligonucleotide probe selected.

Kits and assays of the disclosure may also be provided wherein the oligonucleotide probe is immobilized on a surface. Such a surface may be a bead, a membrane, a column, dipstick, a nanoparticle, the interior surface of a reaction chamber such as the well of a diagnostic plate or inside of a reaction tube, capillary or vessel or the like. The target yeast or fungal organism may be selected from the group consisting of *C*. *albicans, C. glabrata, C. krusei, C. parapsilosis, C. tropicalis C. dubliniensis, C. guilliermondii, C*. *norvegiensis, C. famata, C. haemuloni. C. kefyr, C. utilis, C. viswanathii, A. fumigatus, A. nidulans, A. clavatus, A. niger, A. terreus, A. flavus, A. versicolor* and *Neosartorya fischeri.*

Under these circumstances, the amplification primers and oligonucleotide probes of the disclosure may be designed to a gene specific or genus specific region so as to be able to identify one or more, or most, or substantially all of the desired organisms of the target organism grouping.

The test sample may comprise cells of the target yeast and/or fungal organism. The method may also comprise a step for releasing nucleic acid from any cells of the target yeast or fungal organism that may be present in said test sample. Ideally, the test sample is a lysate of an obtained sample from a patient (such as a swab, or blood, urine, saliva, a bronchial lavage, dental specimen, skin specimen, scalp specimen, transplant organ biopsy, stool, mucus, or discharge sample). The test samples may be a food sample, a water sample, an environmental sample, an end product, end product or in-process industrial sample.

The disclosure also provides for the use of any one of SEQ ID NO.1 to SEQ ID NO.110 in a diagnostic assay for the presence of one or more yeast or fungal species. The species may be selected from the group consisting of C. *albicans, C. glabrata, C. krusei, C. parapsilosis, C*. *tropicalis*,, *C. dubliniensis, C. guilliermondii, C. norvegiensis*, *C. famata, C. haemuloni, C. kefyr, C. utilis, C. viswanathii, A.fumigatus, N.fischeri, A .clavatus, A .niger, A. terreus, A .flavus, A .versicolor and A. nidulans*

The disclosure also provides for kits for use in clinical diagnostics, theranostics, food safety diagnostics, industrial microbiology diagnostics, environmental monitoring, veterinary diagnostics, bio-terrorism diagnostics comprising one or more of the synthetic oligonucleotides of the disclosure. The kits may also comprise one or more articles selected from the group consisting of appropriate sample collecting instruments, reagent containers, buffers, labelling moieties, solutions, detergents and supplementary solutions. The disclosure also provides for use of the sequences, compositions, nucleotide fragments, assays, and kits of the invention in theranostics, Food safety diagnostics, Industrial microbiology diagnostics, Environmental monitoring, Veterinary diagnostics, Bio-terrorism diagnostics.

The nucleic acid molecules, composition, kits or methods may be used in a diagnostic nucleic acid based assay for the detection of yeast and/or fungal species.

The nucleic acid molecules, composition, kits or methods may be used in a diagnostic assay to measure yeast and/or fungal titres in a patient. The titres may be measured *in vitro.*

The nucleic acid molecules, composition, kits or methods may be used in a method of assessing the efficacy of a treatment regime designed to reduce yeast and/or fungal titre in a patient comprising assessing the yeast and/or fungal titre in the patient (by *in vivo* methods or *in vitro* methods) at one or more key stages of the treatment regime. Suitable key stages may include before treatment, during treatment and after treatment. The treatment regime may comprise an antifungal agent, such as a pharmaceutical drug.

The nucleic acid molecules, composition, kits or methods may be used in a diagnostic assay to measure potential yeast and/or fungal contamination, for example, in a hospital. The nucleic acid molecules, composition, kits or methods may be used in the identification and/or characterization of one or more disruptive agents that can be used to disrupt the P2, P2A or P2B gene function. Suitable disruptive agents may be selected from the group consisting of antisense RNA, PNA, siRNA.

### Brief Description of the Drawings

**Figure 1****:** Primers binding sites (grey highlights or bold text or bold and underlined) in P2B of *Candida albicans.*
**Figure 2****:** Primers binding sites in P2 of *Aspergillus fumigatus.* The amplified region of interest is underlined (Position 1 to 326).
**Figure 3****:** Binding site of *C.glabrata* probe P1-CglabP2B (bold and underlined) in the amplified fragment of P2B. PCR primers CglabP2B-F/CglabP2B-R are highlighted.
**Figure 4****:** Binding site of the *A.fumigatus* probe P1-AspP2 (bold and underlined) in the amplified fragment of P2B. PCR primers AspP2-F / AspP2-R are highlighted.
**Figure 5****: Resulting amplification curve from Real-Time PCR assay based on the P2B gene for** *C. glabrata* **with P1-CglabP2B** probe. Specificity of the assay was tested using a panel of DNA from 4 other Candida species and *Aspergillus fumigatus.* The 3 *C*. *glabrata* strains tested were detected and no cross-reaction was observed with DNA from other species.
**Figure 6****. Resulting amplification curve from Real-time PCR based on the P2 gene for** *A fumigatus* **with the P1-AspP2 probe.** Specificity of the assay was tested against a panel of DNA from 6 closely related *Aspergillus* species and *C*. *albicans.* The assay detected *A. fumigatus* but did not detect or cross-react with DNA from *C*. *albicans* or significantly with any of the *Aspergillus* species tested.

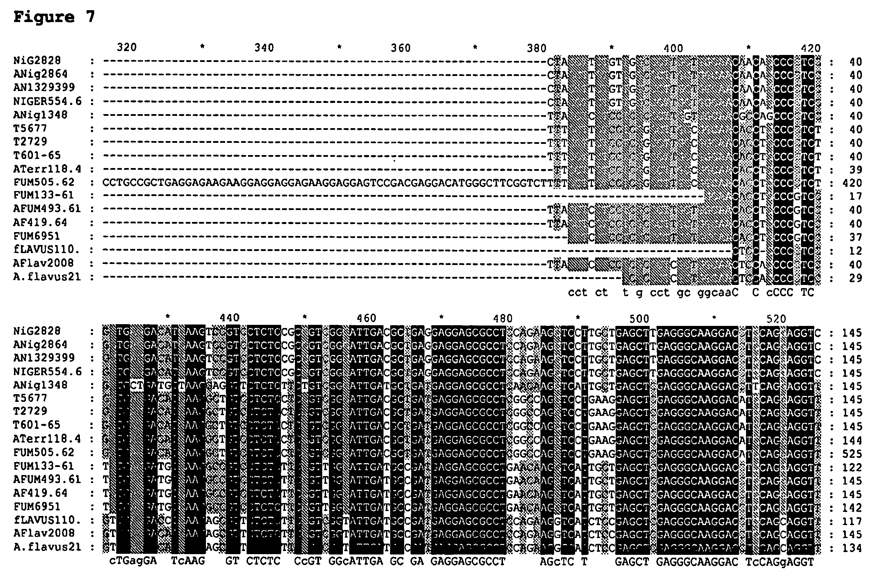

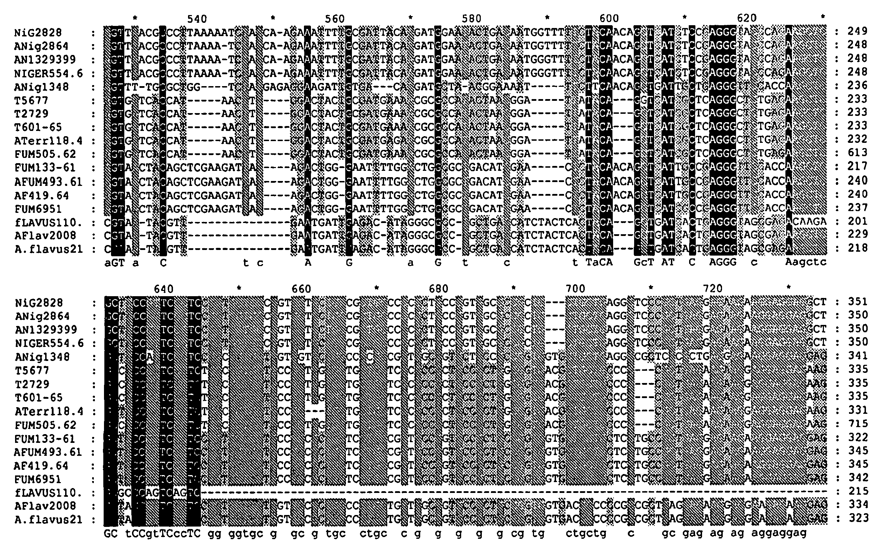

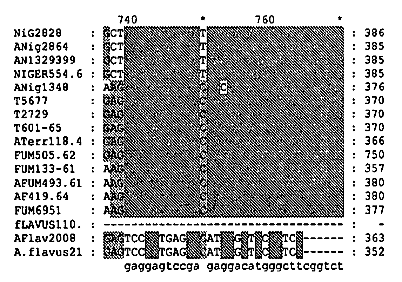

Figure 7. Alignment of sequence information for Aspergillus species.

### Detailed Description of the Invention

### Materials and Methods

### Cell Culture

*Candida* species were cultured in Sabouraud broth (4% wt/vol glucose, 1% wt/vol peptone, 1.5 % agar) for 48 hours at 37°C in a shaking incubator. *Aspergillus* species were cultured in Sabouraud broth (4% wt/vol glucose, 1% wt/vol peptone, 1.5 % agar) or agar for 3-4 days at 25°C.

### DNA Extraction

Cells from *Candida* and *Aspergillus* spp. were pretreated with lyticase or zymolase enzymes prior to DNA isolation. DNA was isolated *Candida* and *Apergillus* spp. using the MagNA Pure System (Roche Molecular Systems) in combination with the MagNA pure Yeast and Bacterial isolation kit III.

DNA sequencing of **P2B/P2** gene regions in *Candida* **and** *Aspergillus* **spp.**

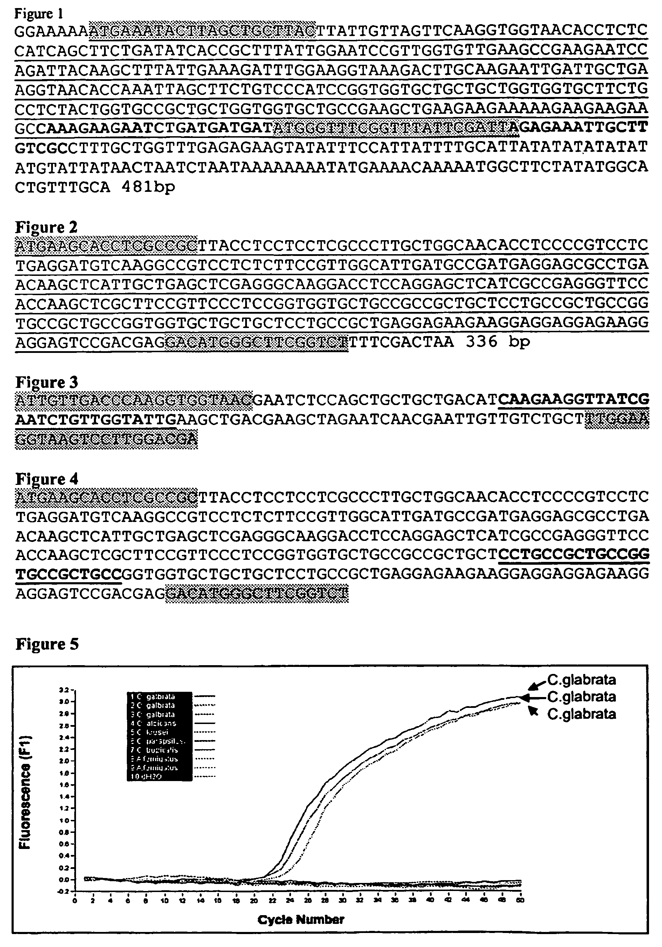

The available sequences of the P2B genes of *Candida* and P2 genes of *Aspergillus spp.* were acquired from the NCBI database and aligned using Clustal W. Three annotated sequences for P2B of *C*. *albicans* (XM_718047.1, XM_717900.1 and AF317662.1) are available in the NCBI database and a sequence of high homology to P2B in *C*. *albicans* has been deposited for *Candida glabrata* (hypothetical protein, XM_444905.1), which is considered here to be P2B. In addition to available sequences for P2 in *Aspergillus* spp., three presumptive P2 sequences are deposited as hypothetical proteins for *Aspergillus spp.* (XM_001213622.1, XM_001397764.1, XM_658508.1). PCR Primers were designed (Table 1) and synthesized by an external company, TibMolBiol, Germany. Primer set CanP2B-F/CanP2B-R was designed to amplify a 335 bp region of the P2B in *Candida* spp. from positions equivalent to bp position 1 to position 335 in *C*. *albicans* (XM_718047.1, XM_717900.1) or position 8 to bp position 342 of the P2B gene in *C*. *albicans* (AF317662.1). Primer set CanP2B-F/CanP2B-R may also in selected Candida species amplify a 326 bp region of the P2A gene. Primer set CanP2B-F/CanP2B flR was designed to amplify a 352 bp region of the P2B equivalent to bp 8 to bp 359 in *C*. *albicans* (AF317662.1). Primer set P2B-F/P2BinR1 were designed to amplify a 312 bp region of the P2B gene from bp position 1 to bp position 312 in XM718047.1/XM 717900.1 and from bp position 8 to bp 319 of AF317662.1. Primer set CglabP2B-F/CglabP2B-R were designed to amplify the region in P2B equivalent to bp position 24 to bp position 158 in *C*. *glabrata* (XM_444905.1). Primer set AspP2-F/AspP2-R were designed to amplify a region of P2 in *Aspergillus* spp. from base pair positions equivalent to 1 to position 326 in *A. fumigatus* (XM_750250.1). Figures 1 and 2 show the positions of the primer set CanP2B-F/CanP2B-R,CanP2B flR and P2BinR1 in *C*. *albicans* and AspP2-F/AspP2-R in *A. fumigatus* respectively. These primer sets were used to amplify regions of the P2B and P2 genes in other *Candida* and *Aspergillus* spp. respectively by conventional PCR on the iCycler BioRad PCR machine or the PTC200 Peltier thermocycler (MJ Research) using the reagents outlined in Table 2 and the thermocycling conditions described in Table 3 or modifications thereof. The PCR reaction products purified with Roche High Pure PCR Product Purification kit or with the ExoSAP-IT kit (USB) according to the manufacturers' instructions were sent for sequencing to Sequiserve, Germany and were sequenced using the forward amplification primer CanP2B-F or AspP2-F. Sequence information was obtained for P2B gene regions for 8 *Candida* spp. (*C*. *albicans, C*. *dubliniensis, C. glabrata, C*. *krusei, C. parapsilosis, C. tropicalis, C. guillermondii, C. lusitanie)* and P2 gene regions for 7 *Aspergillus* species *(A. fumigatus, A. nidulans, A. clavatus, A. niger, A. terreus, A. flavus, A. versicolor)* and *Neosartorya fischeri.*

**Table 1: PCR primers designed for amplification of P2B gene region in Candida and P2 gene region in Aspergillus spp.**

| Primer Name | Primer Sequence 5' to 3' |
|---|---|
| CglabP2B-F | ATTGTTGACCCAAGGTGGTAAC |
| CglabP2B-R | TCGTCCAAGGACTTACCTTCCAA |
| CanP2B-F | ATGAAATACTTAGCTGCTTAC |
| CanP2B-R | TAATCGAATAAACCGAAACCCA |
| CanP2B-flR | GCGACAAGCAATTTCTCT |
| CanP2BinR1 | ATCATCATCAGATTCTTCTTTG |
| AspP2-F | ATGAAGCACCTCGCCGC |
| AspP2-R | AGACCGAAGCCCATGTC |
| P2ForA | GAGGAGCGCCT |
| P2ForB | GAGGAGCGCCTC |
| P2ForC | GGAGGAGCGCCTC |
| P2ForD | TGAGGAGCGCCTC |
| P2RevA | CCGGAGGGAACGGA |
| P2RevB | CCGGAGGGAACGG |
| CAP2BF (For) | ACCTCTCCATCAGCTTCTG |
| CAP2BR (Rev) | TCAGCAATCAATTCTTGC |
| CGP2BF (For) | ACCTCTGTCTTATCATCTGTCG |
| CKP2BR (Rev) | CTCTTCGACGGACTTACC |
| CGP2BF (For) | AAGAAGGTTATCGAATCTGTTG |
| CGP2BR (Rev) | TTCGTCCAAGGACTTACC |
| CTP2BF (For) | TCCGCTTTATTGGAACAAGTTG |
| CTP2BR (Rev) | TTCTTGCAAGTCTTTACC |
| CPP2BF (For) | TCCTCATTGTTGGAATCCGTTG |
| CPP2BR (Rev) | CTCGTTGATGTCTTTACC |

**Table 2: PCR reagents used to amplify P2B gene region in Candida and P2 gene region in Aspergillus spp.**

| **PCR Reaction Mix** | SAMPLE |
|---|---|
| | x 1 |
| 10 x Buffer(100 mM Tris HCl, 15 mM MgCl₂, 500 mM KCl pH 8.3) | 5 µl |
| dNTP's Mix, Roche (10mM dNTP) | 1 µl |
| Primer Forward CanP2B-F or AspP2B-F (10µM) | 1 µl |
| Primer Reverse CanP2B-R CanP2B-flR, Can P2BinR or AspP2B-R (10µM) | 1 µl |
| Polymerase TaqPol, Roche1U/µl | 1 µl |
| H2O, Amgen/Accugene | 36-39 µl |
| Genomic DNA Template | 2- 5 µl |
| TOTAL VOLUME | 50 µl |

**Table 3: PCR reaction conditions used to amplify P2B gene region in Candida and P2 gene region in Aspergillus spp.**

| PCR Thermal profile | | Lid preheating was ON | |
|---|---|---|---|
| Step | Temp | Time | **X 35** |
| 1 | 94°C | 1 min | |
| 2 | 50°C | 1 min | |
| 3 | 72°C | 1 min | |
| 4 | 72°C | 7 min | |
| 5 | 8°C | Hold | |

**Table 4: TaqMan probes (5'-FAM and 3'-BHQ1 labels) based on the P2B/P2 gene for C. glabrata and A. fumigatus.**

| **Probe Name** | **Probe Sequence 5'to 3'** |
|---|---|
| P1-Cglab-P2B | CAAGAAGGTTATCGAATCTGTTGGTATTG |
| P1-AspP2 | CCTGCCGCTGCCGGTGCCGCTGCC |
| P2FumP | ACTACAGCTCGAAGATTA |
| P2FlavP | ACGTTGAATGATTGAGAC |
| P2NigP | TTGCGATTACAAGATGGAA |
| P2TerrP | CTTCGGACTACTGCGATGA |
| CAP2BP | ACCGCTTTATTGGAATCCGTTG |
| CKP2BP | ATCCGACAAGTTAGACAAGTTAATC |
| CGP2BP | AGAATCAACGAATTGTTGTCTGC |
| CTP2BP | ATCTTCCAAATTAGACTTATTGTTGA |
| CPP2BP | GAAGAATCAAGATTATCTACCTTGTTG |

**Table 5: Real-time PCR reagents**

| **Preparation of PCR Reaction Mix** | SAMPLE |
|---|---|
| | x 1 |
| HybProb mix 10 x conc. (LightCycler®FastStartDNA Master HybProbe Kit) | 2 µl |
| MgCl₂ stock solution (Final conc. in reaction is 3 mM) | 1.6 µl |
| Probe P1-CglabP2B or P1-AspP2 | 2 µl |
| Primer Forward CglabP2B-F or AspP2-F | 1 µl |
| Primer Reverse CglabP2B-R or AspP2-R | 1 µl |
| H₂OPCR-grade | 10.4 µl |
| Genomic DNA Template | 2 µl |
| TOTAL VOLUME | 20 µl |

**Table 6: Real-time PCR thermocycling conditions:**

| **PCR Thermal profile** | | | | | |
|---|---|---|---|---|---|
| **Cycle** | **Step** | **Temp** | **Time** | | |
| Activation | 1 | 95°C | 10 min | **X 50** | |
| Amplification | 1 | 95°C | 10 sec | | |
| | 2 | 62 or 65°C | 20 sec | | |
| | 3 | 70°C | 10 sec | | |
| Cooling | 1 | 40°C | Hold | | |
| The PCR was performed with LightCycler® Roche PCR | | | | | |

### Results

### Primer and Probe Design

The publicly available sequence information for the P2B gene in *Candida* spp. was aligned with the newly generated sequence information for the P2B gene in *Candida* spp. and analysed using bioinformatics tools. The sequence information available for the P2 gene in *Aspergillus* spp. was aligned with the newly generated sequence information for the P2 gene in *Aspergillus* spp. and analysed using bioinformatics tools. Species-specific probes were designed based on the compiled P2B and P2 sequence information for *Candida glabrata* and *Aspergillus fumigatus* respectively (Table 4). Figures 3 and 4 show the relative positions of the PCR primers and TaqMan DNA probes for the amplification and detection of *C*. *glabrata* and *A. fumigatus* respectively.

### Real-time PCR:

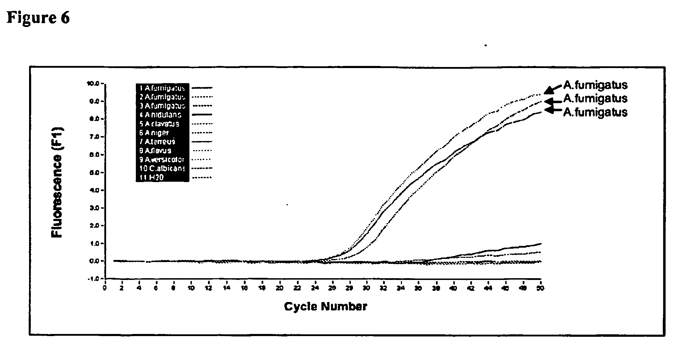

These probes were designed as TaqMan probes and demonstrated for species detection in real-time PCR on the LightCycler using reagents and thermocycling conditions detailed in Tabes 5 and 6. For the C. *glabrata* real-time PCR assay based on the P2B gene, PCR primers CglabP2B-F/CglabP2B-R were combined with TaqMan probe P1-Cglab-P2B. The specificity of the assay for the detection of C. *glabrata* was confirmed by including DNA from a range of closely related Candida species and *A. fumigatus* in the C. *glabrata* real-time PCR assay. The assay detected C. *glabrata* but did not detect or cross-react with DNA from any other *Candida* species tested or with *A. fumigatus* DNA. Figure 5 shows the results of the C. *glabrata* real-time PCR assay and the specificity of the assay for C. *glabrata.* For the *A. fumigatus* real-time PCR assay based on the P2 gene, PCR primers AspP2-F / AspP2-R were combined with TaqMan probe P1-AspP2. The specificity of the assay for the detection of *A. fumigatus* was confirmed by including DNA from a range of closely related Aspergillus species and *C. albicans* in the *A. fumigatus* real-time PCR assay. The assay detected *A. fumigatus* but did not detect or cross-react with DNA from *C*. *albicans* or any of the four *Aspergillus* species tested. Two additional *Aspergillus* spp. did show a small amount of cross reaction in the *A. fumigatus* real-time PCR assay but this can be eliminated with further assay optimisation. Figure 6 shows the results of the *A. fumigatus* real-time PCR assay.

In so far as any sequence disclosed herein differs from its counterpart in the attached sequence listing in PatentIn3.3 software, the sequences within this body of text are to be considered as the correct version.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### SEQ IDs

Sites of probes, oligonucleotides etc. are shown in bold and underlined.

N or x= any nucleotide; w=a/t, m=a/c, r=a/g, k=g/t, s=c/g, y=c/t, h=a/t/c, v=a/g/c, d=a/g/t, b=g/t/c. In some cases, specific degeneracy options are indicated in parenthesis: e.g.: (a/g) is either A or G.
**SEQ ID NO 1:** CglabP2B-F
ATTGTTGACCCAAGGTGGTAAC
**SEQ ID NO 2:** CglabP2B-R
TCGTCCAAGGACTTACCTTCCAA
**SEQ ID NO 3:** CanP2B-F
ATGAAATACTTAGCTGCTTAC
**SEQ ID NO 4:** CanP2B-R
TAATCGAATAAACCGAAACCCA
**SEQ ID NO 5:** Can P2Bf1R1
GCGACAAGCAATTTCTCT
**SEQ ID NO 6:** Can P2BinR1
ATCATCATCAGATTCTTCTTTG
**SEQ ID NO 7:** AspP2-F
ATGAAGCACCTCGCCGC
**SEQ ID NO 8:** AspP2-R
AGACCGAAGCCCATGTC
**SEQ ID NO 9:** P1-Cglab-P2B
CAAGAAGGTTATCGAATCTGTTGGTATTG
**SEQ ID NO 10:** P1-AspP2
CCTGCCGCTGCCGGTGCCGCTGCC
**SEQ ID NO 11:**
>glab2897\(PF) sequences generated for C.glabrata 
CTATTGTTGACCCAAGGTGGTAACGAATCTCCAGCTGCTGCTGACATCAAGA
AGGTTATCGAATCTGTTGGTATTGAAGCTGACGAAGCCAGAATCAACGAAT
TGTTGTCTGCTTTGGAAGGTAAGTCCTTGGACGAATTGATCGCTGAAGGTCA
ACAAAAGTTCGCCTCTGTTCCAGTTGGTGGTGCTGCTGCTGGTGGTGCTTCC
GCTGCTGCTGGTGGTGCCGCTGCCGGTGAAGCCGCTGAAGAAAAGGAAGAA
GAAGCTGCTGAAGAATCCGATGACGACATGGGTTTCGGTTTATTCGATTA
 **SEQ ID NO 12:**
>glab8018\(PF) sequences generated for C.glabrata 
TTGTTGACCCAAGGTGGTAACGAATCTCCAGCTGCTGCTGACATCAAGAAG
GTTATCGAATCTGTTGGTATTGAAGCTGACGAAGCCAGAATCAACGAATTGT
TGTCTGCTTTGGAAGGTAAGTCCTTGGACGAATTGATCGCTGAAGGTCAACA
AAAGTTCGCCTCTGTTCCAGTTGGTGGTGCTGCTGCTGGTGGTGCTTCCGCT
GCTGCTGGTGGTGCCGCTGCCGGTGAAGCCGCTGAAGAAAAGGAAGAAGA
AGCTGCTGAAGAATCCGATGACGACATGGGTTTCGGTTTATTCGATTA
 **SEQ ID NO 13:**
>glab4692\(PF) sequences generated for C.glabrata 
CTATTGTTGACCCAAGGTGGTAACGAATCTCCAGCTGCTGCTGACATCAAGA
AGGTTATCGAATCTGTTGGTATTGAAGCTGACGAAGCCAGAATCAACGAAT
TGTTGTCTGCTTTGGAAGGTAAGTCCTTGGACGAATTGATCGCTGAAGGTCA
ACAAAAGTTCGCCTCTGTTCCAGTTGGTGGTGCTGCTGCTGGTGGTGCTTCC
GCTGCTGCTGGTGGTGCCGCTGCCGGTGAAGCCGCTGAAGAAAAGGAAGAA
GAAGCTGCTGAAGAATCCGATGACGACATGGGTTTCGGTTTATTCGATTA
 **SEQ ID NO 14:**
>glab138\(PF) sequences generated for C.glabrata 
CTATTGTTGACCCAAGGTGGTAACGAATCTCCAGCTGCTGCTGACATCAAGA
AGGTTATCGAATCTGTTGGTATTGAAGCTGACGAAGCTAGAATCAACGAAT
TGTTGTCTGCTTTGGAAGGTAAGTCCTTGGACGAATTGATCGCTGAAGGTCA
ACAAAAGTTCGCCTCTGTTCCAGTTGGTGGTGCTGCTGCTGGTGGTGCTTCC
GCTGCTGCTGGTGGTGCCGCTGCCGGTGAAGCTGCTGAAGAAAAGGAAGAA
GAAGCTGCTGAAGAATCCGATGACGACATGGGTTTCGGTTTATTCGATTA
 **SEQ ID NO 15:**
>krusei3165\(P2BF) sequence generated for C. krusei 
TTACTCTTAGTCAATGCTGGTAAAACCGCACCATCTGCTGCAGATGTTACCT
CTGTCTTATCATCTGTCGGTATCGAAGTTGAATCCGACAAGTTAGACAAGTT
AATCTCCGAATTAGAAGGTAAGTCCGTCGAAGAGTTGATTGCTGAAGGTAC
TGAAAAGATGGCTTCTGCTCCAGGTGCAGCAGCTGCTCCAGCTTCTGGTGCA
GGTGCTTCCACCGAATCTGCTGCAGCAGAAGAAGTTGAAGAAGAAAAGGAA
GAATCCGATGATGACATGGGTTTCGGTTTATTCGATTA
 **SEQ ID NO 16:**
>krusei9560\(P2BF) sequence generated for C. krusei 
CATCTGCTGCAGATGTTACCTCTGTCTTATCATCTGTCGGTATCGAAGTTGA
ATCCGACAAGTTAGACAAGTTAATCTCCGAATTAGAAGGTAAGTCCGTCGA
AGAGTTGATTGCTGAAGGTACTGAAAAGATGGCTTCTGCTCCAGGTGCAGC
AGCTGCTCCAGCTTCTGGTGCAGGTGCTTCCACCGAATCTGCTGCAGCAGAA
GAAGTTGAAGAAGAAAAGGAAGAATCCGATGATGACATGGGTTTCGGTTTA
TTCGATTA
 **SEQ ID NO 17:**
>krusei6055\(P2BF) sequence generated for C. krusei 
CATCTGCTGCAGATGTTACCTCTGTCTTATCATCTGTCGGTATCGAAGTTGA
ATCCGACAAGTTAGACAAGTTAATCTCCGAATTAGAAGGTAAGTCCGTCGA
AGAGTTGATTGCTGAAGGTACTGAAAAGATGGCTTCTGCTCCAGGTGCAGC
AGCTGCTCCAGCTTCTGGTGCAGGTGCTTCCACCGAATCTGCTGCAGCAGAA
GAAGTTGAAGAAGAAAAGGAAGAATCCGATGATGACATGGGTTTCGGTTTA
TTCGATTA
 **SEQ ID NO 18:**
>krusei573\(P2B) sequence generated for C. krusei 
TTACTCTTAGTACATGCTGGTAAAACCGCACCATCTGCTGCAGATGTTACCT
CTGTCTTATCATCTGTCGGTATCGAAGTTGAATCCGACAAGTTAGACAAGTT
AATCTCCGAATTAGAAGGTAAGTCCGTCGAAGAGTTGATTGCTGAAGGTAC
TGAAAAGATGGCTTCTGCTCCAGGTGCAGCAGCTGCTCCAGCTTCTGGTGCA
GGTGCTTCCACCGAATCTGCTGCAGCAGAAGAAGTTGAAGAAGAAAAGGAA
GAATCCGATGATGACATGGGTTTCGGTTTATTCGATTA
 **SEQ ID NO 19:**
>CA562P2B sequence generated for C.albicans 
TTATTGTTAGTTCAAGGTGGTAACACCTCTCCATCAGCTTCTGATATCACCG
CTTTATTGGAATCCGTTGGTGTTGAAGCCGAAGAATCCAGATTACAAGCTTT
ATTGAAAGATTTGGAAGGTAAAGACTTGCAAGAATTGATTGCTGAAGGTAA
CACCAAATTAGCTTCTGTCCCATCCGGTGGTGCTGCTGCTGGTGGTGCTTCT
GCCTCTGCTGGTGCCGCTGCTGGTGGTGCTGCTGAAGCTGAAGAAGAAAAA
GAAGAAGAAGCCAAAGAAGAATCTGATGATGATATGGGTTTCGGTTTATTC
GATTAGAGAAATTGCTTGTCGC
 **SEQ ID NO 20:**
>A765\ (P2BF) P2B sequence generated for C.albicans 
TTATTGTTAGTTCAAGGTGGTAACACCTCTCCATCAGCTTCTGATATCACCG
CTTTATTGGAATCCGTTGGTGTTGAAGCCGAAGAATCCAGATTACAAGCTTT
ATTGAAAGATTTGGAAGGTAAAGACTTGCAAGAATTGATTGCTGAAGGTAA
CACCAAATTAGCTTCTGTCCCATCCGGTGGTGCTGCTGCTGGTGGTGCTTCT
GCCTCTGCTGGTGCCGCTGCTGGTGGTGCTGCTGAAGCTGAAGAAGAAAAA
GAAGAAGAAGCCAAAGAAGAATCTGATGATGATATGGGTTTCGGTTTATTC
GATTAGAGAAATTGCTTGTCGC
 **SEQ ID NO 21:**
>A3156\ (P2BF) P2B sequence generated for C.albicans 
TTATTGTTAGTTCAAGGTGGTAACACCTCTCCATCAGCTTCTGATATCACCG
CTTTATTGGAATCCGTTGGTGTTGAAGCCGAAGAATCCAGATTACAAGCTTT
ATTGAAAGATTTGGAAGGTAAAGACTTGCAAGAATTGATTGCTGAAGGTAA
CACCAAATTAGCTTCTGTCCCATCCGGTGGTGCTGCTGCTGGTGGTGCTTCT
GCCTCTGCTGGTGCCGCTGCTGGTGGTGCTGCTGAAGCTGAAGAAGAAAAA
GAAGAAGAAGCCAAAGAAGAATCTGATGATGATATGGGTTTCGGTTTATTC
GATTAGAGAAATTGCTTGTCGC
 **SEQ ID NO 22:**
>A2700\ (P2BF) P2B sequence generated for C.albicans TTATTGTTAGTTCAAGGTGGTAACACCTCTCCATCAGCTTCTGATATCACCG 
CTTTATTGGAATCCGTTGGTGTTGAAGCYGAAGAATCCAGATTACAAGCTTT
ATTGAAAGATTTGGAAGGTAAAGACTTGCAAGAATTGATTGCTGAAGGTAA
CACCAAATTAGCTTCTGTCCCATCCGGTGGTGCTGCTGCTGGTGGTGCTTCT
GCCTCTRCTGGTGCCGCTGCTGGYGGTGCTGCYGAAGCTGAAGAAGAAAAA
GAAGAAGAAGCCAAAGAAGAATCTGATGATGATATGGGTTTCGGTTTATTC
GATTAGAGAAATTGCTTGTCGC
 **SEQ ID NO 23:**
>D3999\ (P2BF) P2B sequence generated for C.dubliniensis 
TTGTTGTTAGTTCAAGGTGGTAACGCCACTCCATCAGCTTCTGATATCAGCG
CTGTCTTGGAAACTGTTGGTGTTGAAGCCGAAGAATCCAGATTACAAGCTTT
ATTGAAAGATTTGGAAGGTAAAGATTTGCAAGAATTGATTGCTGAAGGTAA
CACCAAATTAGCTTCTGTCCCAACCGGTGGTGCTGCTGCTGGTGGTGCTTCC
GGTTCTGCTGGTGCCGCTTCTGGTGCCGCTGCTGAAGCTGAAGAAGAAAAA
GAAGAAGAAGCTAAAGAAGAATCTGATGATGATATGGGTTTCGGTTTATTC
GATTAGAGAAATTGCTTGTCGC
 **SEQ ID NO 24:**
>D7987\(P2BF) sequence generated for C.dubliniensis 
TTGTTGTTAGTTCAAGGTGGTAACGCCACTCCATCAGCTTCTGATATCAGCG
CTGTCTTGGAAACTGTTGGTGTTGAAGCCGAAGAATCCAGATTACAAGCTTT
ATTGAAAGATTTGGAAGGTAAAGATTTGCAAGAATTGATTGCTGAAGGTAA
CACCAAATTAGCTTCTGTCCCAACCGGTGGTGCTGCTGCTGGTGGTGCTTCC
GGTTCTGCTGGTGCCGCTTCTGGTGCCGCTGCTGAAGCTGAAGAAGAAAAA
GAAGAAGAAGCCAAAGAAGAATCTGATGATGATA
 **SEQ ID NO 25:**
>CP 604\(CanP2B-FOR>><<REV) P2B sequences generated for C.parapsilosis 
ATGAAATACTTAGCTGCTTACTTATTATTGGTCCAAGGTGGTAACGCCTCCC
CATCTGCTTCAGACATCTCCTCATTGTTGGAATCCGTTGGTGTTGAAGTTGA
AGAATCAAGATTATCTACCTTGTTGAAAGACTTGGAAGGTAAAGACATCAA
CGAGTTGATTGCTGAAGGTAACACCAAATTGGCCTCAGTTCCATCTGGTGGT
GCTGCTGTTGCTTCCGGTTCTGGTGCTTCTGGTGCCGCTGCTGGTGGTGCTGC
TGAAGAAGCTAAGGAAGAAGCCAAGGAAGAAGAAAAGGAAGAATCTGATG
ATGACATGGGTTTCGGTTTATTCGATTA
 **SEQ ID NO 26:**
>P2194\(P2BF) sequences generated for C.parapsilosis 
TTATTATTGGTCCAAGGTGGTAACGCCTCCCCATCTGCTTCAGACATCTCCTC
ATTGTTGGAATCCGTTGGTGTTGAAGTTGAAGAATCAAGATTATCTACCTTG
TTGAAAGACTTGGAAGGTAAAGACATCAACGAGTTGATTGCTGAAGGTAAC
ACCAAATTGGCCTCAGTTCCATCTGGTGGTGCTGCTGTTGCTTCCGGTTCTG
GTGCTTCTGGTGCCGCTGCTGGTGGTGCTGCTGAAGAAGCTAAGGAAGAAG
CCAAGGAAGAAGAAAAGGAAGAATCTGATGATGACATGGGTTTCGGTTTAT
TCGATTA
 **SEQ ID NO 27:**
>P96141\(P2BF) sequences generated for C.parapsilosis 
GGTGGTAACGCCTCCCCATCCGCTTCAGACATCTCATCCTTGTTGGAATCCG
TTGGTGTTGAAGTTGAAGAATCAAGATTGTCCCTCTTGTTGAAAGACTTGGA
AGGTAAAGACATCAACGAATTGATTGCTGAAGGTAACACCAAGTTGGCTTC
AGTTCCAACTGGTGGTGCTGCTGTTGCTTCTGGTTCTGGTGCTTCAGGTGCC
GCTGCTGGTGGTGCTGCTGAAGAAGCCAAAGAAGAATCTGATGATGAT
 **SEQ ID NO 28:**
>CT94\(P2BF) sequences generated for C.tropicalis 
GGTGGTAACGCTTCCCCATCTGCTTCTGACATCTCCGCTTTATTGGAACAAG
TTGGTGCTGAAGTTGAATCTTCCAAATTAGACTTATTGTTGAAAGAATTGGA
AGGTAAAGACTTGCAAGAATTGATTGCCGAAGGTAACACTAAATTCGCCTC
TGTCCCATCCGGTGGTGCTGCTGCTGCTTCYTCTGGTTCCGCTGCCGCTGCTG
GTGGTGCCGCTGCCGAAGCTGAAGAAGAAAAAGAAGAAGAAGCCAAAGAA
GAATCTGATGATGAT
 **SEQ ID NO 29:**
>CT2311\(P2BF) sequences generated for C.tropicalis 
GGTGGTAACGCTTCCCCATCTGCTTCTGACATCTCCGCTTTATTGGAACAAG
TTGGTGCTGAAGTTGAATCTTCCAAATTAGACTTATTGTTGAAAGAATTGGA
AGGTAAAGACTTGCAAGAATTGATTGCCGAAGGTAACACTAAATTCGCCTC
TGTCCCATCCGGTGGTGCTGCTGCTGCTTCCTCTGGTTCCGCTGCCGCTGCTG
GTGGTGCCGCTGCCGAAGCTGAAGAAGAAAAAGAAGAAGAAGCCAAAGAA
GAATCTGATGATGAT
 **SEQ ID NO 30:**
>T-8157\(PF) sequences generated for C.tropicalis 
TTATTATTAGTCCAAGGTGGTAACRCTTCCCCATCTGCTTCTGACATCTCCGC
TTTATTGGAACAAGTTGGTGCTGAAGTTGAATCTTCCAAATTAGACTTATTG
TTGAAAGAATTGGAAGGTAAAGACTTGCAAGAATTGATTGCCGAAGGTAAC
ACTAAATTCGCCTCTGTCCCATCCGGTGGTGCTGCYGCTGCTTCCTCTGGTTC
CGCTGCCGCTGCTGGTGGTGCCGCTGCCGAAGCTGAAGAAGAAAAAGAAGA
AGAAGCCAAAGAAGAATCTGATGATGAT
 **SEQ ID NO 31:**
>T-2424\(PF) sequences generated for C.tropicalis 
TTATTATTAGTCCAAGGTGGTAACGCTTCCCCATCTGCTTCTGACATCTCCGC
TTTATTGGAACAAGTTGGTGCTGAAGTTGAATCTTCCAAATTAGACTTATTG
TTGAAAGAATTGGAAGGTAAAGACTTGCAAGAATTGATTGCCGAAGGTAAC
ACTAAATTCGCCTCTGTCCCATCCGGTGGTGCTGCTGCTGCTTCCTCTGGTTC
CGCTGCCGCTGCTGGTGGTGCCGCTGCCGAAGCTGAAGAAGAAAAAGAAGA
AGAAGCCAAAGAAGAATCTGATGATGAT
 **SEQ ID NO 32:**
>T-3895\(PF) sequences generated for C.tropicalis 
TTATTATTAGTCCAAGGTGGTAACGCTTCCCCATCTGCTTCTGACATCTCCGC
TTTATTGGAACAAGTTGGTGCTGAAGTTGAATCTTCCAAATTAGACTTATTG
TTGAAAGAATTGGAAGGTAAAGACTTGCAAGAATTGATTGCCGAAGGTAAC
ACTAAATTCGCCTCTGTCCCATCCGGTGGTGCTGCTGCTGCTTCTTCTGGTTC
 
CGCTGCCGCTGCTGGTGGTGCCGCTGCCGAAGCTGAAGAAGAAAAAGAAGA
AGAAGCCAAAGAAGAATCTGATGATGAT
 **SEQ ID NO 33:**
>guill2672\(P2BF) sequences generated for C.guilliermondii 
TTRTTGTTGGTKRACGCCGGTAACACCTCCCCATCTGCTGCTGACATCAAGG
CTGTCTTGGAGTCGGTTTCCATAGAAGTTGACGACGAGAAGGTGTCCAAGTT
GTTGAGCGAAGTTGAGGGAAAGAATGCTGAAGAATTGATCGCTGAAGGTAA
CGAAAAATTGTCTTCTGTTCCAACTGGTGGACCAGCTGCTGCTTCCTCTGGA
TCTGCTGCCGCTGCCGATGCTCCTGCTGCCGAAGAGGCCGCTGAGGAGGCC
GCTGAGGAGTCTGACGACGACATGGGTTTCGGTTTATTCGATTA
 **SEQ ID NO 34:**
>guill6021\(P2BF) sequences generated for C.guilliennondii 
TTRTTGTTGGTKRACGCCGGTAACACCTCCCCATCTGCTGCTGACATCAAGG
CTGTCTTGGAGTCGGTTTCCATAGAAGTTGACGACGAGAAGGTGTCCAAGTT
GTTGAGCGAAGTTGAGGGAAAGAATGCTGAAGAATTGATCGCTGAAGGTAA
CGAAAAATTGTCTTCTGTTCCAACTGGTGGACCAGCTGCTGCTTCCTCTGGA
TCTGCTGCCGCTGCCGATGCTCCTGCTGCCGAAGAGGCCGCTGAGGAGGCC
GCTGAGGAGTCTGACGACGACATGGGTTTCGGTTTATTCGATTA
 **SEQ ID NO 35:**
>lus7270\(P2BF) sequences generated for C.lusitanie 
TTATTGTTGGTCAACGCTGGTAACACCGCCCCATCTGCTGCTGACGTCAAGA
AGGTCTTGGAATCCGTCTCTATTGAGGTTGAGGACGACAAGGTTGAGAAGT
TGTTGGCTGAAGTTGAAGGCAAGAACGTCGAAGAGTTGATTGCCGAGGGTA
ACGAGAAGTTGTCTTCTGTTCCATCTGGTGCTCCAGCTGCTGCTGGTGCCGC
TGCTGCTTCTGGTTCTACTGAGGCTGCTGCTGAAGAGCCACAAGAAGAAGA
GAAGGAGGAGTCTGACGACGACATGGGTTTCGGTTTATTCGATTA
 **SEQ ID NO 36:**
>AF419.64\(AspP2-F) P2 sequences generated for A.fumigatus 
TTACCTCCTCCTCGCCCTTGCTGGCAACACCTCCCCGTCCTCTGAGGATGTCA
AGGCCGTCCTCTCTTCCGTTGGCATTGATGCCGATGAGGAGCGCCTGAACAA
GCTCATTGCTGAGCTCGAGGGCAAGGACCTCCAGGAGGTTAGTAACTACAG
CTCGAAGATTACAGACTGGGAATTTTGGACTGGCGCTGACATCGAACTCTAC
AACAGCTCATTGCCGAGGGTTCCACCAAGCTCGCTTCCGTTCCCTCCGGTGG
TGCTGCCGCCGCTGCTCCTGCCGCTGCCGGTGCCGCTGCCGGTGGTGCTGCT
GCTCCTGCCGCTGAGGAGAAGAAGGAGGAGGAGAAGGAGGAGTCCGACGA
GGACATGGGCTTCGGTCT
 **SEQ ID NO 37:**
>F-6951\(APF) P2 sequences generated for A.fumigatus 
CCTCCTCCTCGCCCTTGCTGGCAACACCTCCCCGTCCTCTGAGGATGTCAAG
GCCGTCCTCTCTTCCGTTGGCATTGATGCCGATGAGGAGCGCCTGAACAAGC
TCATTGCTGAGCTCGAGGGCAAGGACCTCCAGGAGGTTAGTAACTACAGCT
CGAAGATTACAGACTGGGAATTTTGGACTGGCGCTGACATCGAACTCTACA
ACAGCTCATCGCCGAGGGTTCCACCAAGCTCGCTTCCGTTCCCTCCGGTGGT
GCTGCCGCCGCTGCTCCTGCCGCTGCCGGTGCCGCTGCCGGTGGTGCTGCTG
 
CTCCTGCCGCTGAGGAGAAGAAGGAGGAGGAGAAGGAGGAGTCCGACGAG
GACATGGGCTTCGGTCT
 **SEQ ID NO 38:**
>F-133-61\(APF) P2 sequences generated for A.fumigatus 
GCAACACCTCCCCGTCCTCTGAGGATGTCAAGGCCGTCCTCTCTTCCGTTGG
CATTGATGCCGATGAGGAGCGCCTGAACAAGCTCATTGCTGAGCTCGAGGG
CAAGGACCTCCAGGAGGTTAGTAACTACAGCTCGAAGATTACAGACTGGGA
ATTTTGGACTGGCGCTGACATCGAACTCTACAACAGCTCATTGCCGAGGGTT
CCACCAAGCTCGCTTCCGTTCCCTCCGGTGGTGCTGCCGCCGCTGCTCCTGC
CGCTGCCGGTGCCGCTGCCGGTGGTGCTGCTGCTCCTGCCGCTGAGGAGAA
GAAGGAGGAGGAGAAGGAGGAGTCCGACGAGGACATGGGCTTCGGTCT
 **SEQ ID NO 39:**
>AF493.61\(Asp2F) P2 sequences generated for A.fumigatus 
TTACCTCCTCCTCGCCCTTGCTGGCAACACCTCCCCGTCCTCTGAGGATGTCA
AGGCCGTCCTCTCTTCCGTTGGCATTGATGCCGATGAGGAGCGCCTGAACAA
GCTCATTGCTGAGCTCGAGGGCAAGGACCTCCAGGAGGTTAGTAACTACAG
CTCGAAGATTACAGACTGGGAATTTTGGACTGGCGCTGACATCGAACTCTAC
AACAGCTCATTGCCGAGGGTTCCACCAAGCTCGCTTCCGTTCCCTCCGGTGG
TGCTGCCGCCGCTGCTCCTGCCGCTGCCGGTGCCGCTGCCGGTGGTGCTGCT
GCTCCTGCCGCTGAGGAGAAGAAGGAGGAGGAGAAGGAGGAGTCCGACGA
GGACATGGGCTTCGGTCT
 **SEQ ID NO 40:**
>AF1085-P2\(AspP2-F) P2 sequences generated for N.fischeri 
TTACCTCCTCCTCGCCCTTGCTGGCAACACCTCCCCCTCCGCTGAGGATGTC
AAGGCCGTCCTCTCTTCCGTCGGCATTGACGCCGATGAGGAGCGCCTGAAC
AAGCTCATTGCTGAGCTCGAGGGCAAGGACCTCCAGGAGGTTAGTACACAC
GGCTTGAATATTACCGACTGAGAATTTTGGACCGGCGCTGACATCGATTTCT
ACAACAGCTGATCGCTGAGGGTTCCGCCAAGCTCGCTTCCGTTCCCTCCGGT
GGTGCCGGTGGTGCCGCTGCTCCTGCCGCTGGCGGTGCCGCTGCCGGTGGTG
CTGCTGCCGCTCCCGCCGAAGAGAAGGAGGAGGAGAAGGAGGAGTCCGAC
GAGGACATGGGCTTCGGTCT
 **SEQ ID NO** 41:
>Nid100-2\(P2F) P2B sequence for A. nidulans 
CTACCTCCTCCTCGCCCTTGCTGGCAACGAGTCTCCCTCCGCCTCCGACATC
AAGGAGGTTCTCTCTTCCGTCGGTGTTGACGCCGACGACGAGCGCCTCGAG
AAGCTCATTGCTGAGCTCCAGGGCAAGGACATCAACGAGGTTCGTTATTGC
ATATAGAGTTGGAAGACGCGGACTGCGGGCTAACGATAATCTTTAAACAGC
TGATCGCTGAGGGTACCACCAAGCTTGCCTCCGTTCCCTCCGGCGGTGCTGG
TGGTGCTGCCCCTGCTGCCGCTGCCGGTGGTGCTGCTGCTGCCGAGG
CCCCCGCTGCTGAGAAGGAGGAGGAGAAGGAGGAGTCCGATGAGGACATG
GGCTTCGGTCT
 **SEQ ID NO 42:**
>Nid7063\(P2F) P2B sequence for A. nidulans CCTTGCTGGCAACGAGTCTCCCTCCGCCTCCGACATCAAGGAGGTTCTCTCT 
TCCGTCGGTGTTGACGCCGACGACGAGCGCCTCGAGAAGCTCATTGCTGAG
CTCCAGGGCAAGGACATCAACGAGGTTCGTTATTGCATAGGGTTGGAAGAC
GCGGACAGCGGGCTAACGATAATCTTCTGAACAGCTGATCGCTGAGGGTAC
CACCAAGCTTGCCTCCGTTCCCACCGGCGGTGCTGGTGCTGCTGCCCCTGCT
GCCGCTGCCGGTGGTGCTGCCGCTGCCGAGGCTCCCGCTGCTGAGAAGGAG
GAGGAGAAGGAGGAGTCCGATGAGGACATGGGCTTCGGTCT
 **SEQ ID NO 43:**
>AC5138\(AspP2-F) P2 sequences generated for A.clavatus 
TTACCTCCTCCTCGCCCTTGGTGGCAACGCCAGCCCCTCCGCTGCTGATGTT
AAGGAGGTTCTCTCTTCTGTCGGCATTGATGCTGATGAGGAGCGCCTCAACA
AGCTCATTGCTGAGCTCGAGGGCAAGGACCTTCAGGAGGTTAGTTTTGCGCT
GGTCTACGAGAGGAAGATTGTGACAAGATGCTAACGGAAAATTTCTTCAAC
AGCTGATTGCTGAGGGTTCCACCAAGCTCGCTTCCATTCCCTCCGGCGGTGC
TGGTGGTGCTGCCCCCGCCGCTGGCGGTGCTGCCGCCGGTGGTGCTGCTGAG
GCCGCTCCCGCTGAGGAGAAGGAGGAGGAGAAGGAGGAGTCCGACGACGA
CATGGGCTTCGGTCT
 **SEQ ID NO 44:**
>AN1329399-P2\(AspP2-F) P2 sequences generated for A.niger 
CTACCTTCTGTTGGCCCTTGCTGGCAACAACACCCCCTCCGCTGAGGACATC
AAGTCCGTCCTCTCCGCCGTCGGCATTGACGCTGAGGAGGAGCGCCTCCAG
AAGCTCCTTGCTGAGCTTGAGGGCAAGGACCTCCAGGAGGTCAGTTAACGC
CCTTAAAATCTACCAAGAAATTTTGCGATTACAAGATGGAATACTGACAAT
GGGTTTTCTACAACAGCTCATCTCCGAGGGTACCCAGAAGCTCGCTTCCGTT
CCCTCCGGTGGTGCCGGTGCTGCTGCCGCTGCCCCCGCTGCCGGTGGCGCCG
CTGCTGCTGAGGCTCCCGCTGAGGAGAAGAAGGAGGAGGCTGCTGAGGAGT
CCGATGAGGACATGGGCTTCGGTCT
 **SEQ ID NO 45:**
>AN2864\(Asp2F) sequences generated for A.niger 
CTACCTTCTGTTGGCCCTTGCTGGCAACAACACCCCCTCCGCTGAGGACATC
AAGTCCGTCCTCTCCGCCGTCGGCATTGACGCTGAGGAGGAGCGCCTCCAG
AAGCTCCTTGCTGAGCTTGAGGGCAAGGACCTCCAGGAGGTCAGTTAACGC
CCTTAAAATCTACCAAGAAATTTTGCGATTACAAGATGGAATACTGACAAT
GGTTTTCTACAACAGCTCATCTCCGAGGGTACCCAGAAGCTCGCTTCCGTT
CCCTCCGGTGGTGCCGGTGCTGCTGCCGCTGCCCCCGCTGCCGGTGGCGCCG
CTGCTGCTGAGGCTCCCGCTGAGGAGAAGAAGGAGGAGGCTGCTGAGGAGT
CCGATGAGGACATGGGCTTCGGTCT
 **SEQ ID NO 46:**
>AT118.46 \(AspP2 -F) P2 sequences generated for A.terreus 
TTCCTTCTCCTCGGCCTTGCCGGCAACACCTCCCCCTCTGCTGAGGACATCA
AGGCTGTCCTCTCCTCCGTCGGCATTGACGCTGATGAGGAGCGCCTCGGCCA
GCTCCTGAAGGAGCTCGAGGGCAAGGACATCCAGGAGGTTAGTGATCACCA
TAACTTCGGACTACTGCGATGAGAACGCGCCATACTAACGGATTATACAGC
TCATCGCTCAGGGCTCTGAGAAGCTCGCTTCCGTTCCCTCTGGCGGTGCTGC
CGCTGCTGCTGCTCCCGCCGCTGCCGCTGGCGGTGACGCTGCTGCCCCCGCT
 
GAGAAGAAGGAGGAGGAGAAGGAGGAGGAGTCCGACGAGGACATGGGCTT
CGGTCT
 **SEQ ID NO 47:**
>AF2008 \(AspP2 -F) P2 sequences generated for A.flavus 
TTACCTCCTCCTCGCCCTCGCTGGCAACTCCACCCCCTCCGTTGAGGACATC
AAGAGCGTTCTCTCTTCCGTCGGTATTGATGCCGATGAGGAGCGCCTCCAGA
AGGTCATCTCCGAGCTCGAGGGCAAGGACCTCCAGCAGGTTCGTAATACGT
TGAATGATTGAGACATAGGGCGCCTGCTGACCATCTACTCACTACAGCTGAT
CACTGAGGGTAGCGAGAAGCTCGCTACCGTTCCCTCCGGTGGTGCTGGTGCC
GCTGCCCCTGCTGCTGGCGGTGCCGCTGCCGGTGGTGACGCCCCCGCCGCTG
AGGAGAAGGAGGAAGAGAAGGAGGAGTCCGATGAGGACATGGGCTTCGGT
CT
 **SEQ ID NO 48:**
>AV1323 \(AspP2 -F) P2 sequences generated for A.versicolor 
TCGCCCTTGCTGGCAACGAGAGCCCCTCTGCTTCCGACATTAAGGAGGTTCT
GTCCTCCGTCGGTGTTGACGCTGACAACGAGCGCCTCGAGAAGCTCATCGCT
GAGCTCCAGGGCAAGGACATCAACGAGGTTCGTTTTGACAGATGCATTTGA
AATACTTGGCCAGCAGACTAATGAAACCTCTTCTGCAGTTGATCGCTGAGGG
TACCACCAAGCTCGCTTCCGTTCCCTCTGGCGGTGGTGGTGGTGCTGCCGCC
CCCGCTGCTGGTGGCGCTGCCGCCGCTGAGGCCCCTGCTGCTGAGAAGGAG
GAGGAGAAGGAGGAGTCCGACGAGGACATGGGCTTCGGTCT
 **SEQ ID NO 49:**
>AV2916\(Asp2F) sequences generated for A.versicolor 
CCCTTGCTGGCAACGAGAGCCCCTCTGCTTCCGACATTAAGGAGGTTCTGTC
CTCCGTCGGTGTTGACGCTGACGACGAGCGCCTCGAGAAGCTCATCGCTGA
GCTCCAGGGCAAGGACATCAACGAGGTTCGTTTGACAGATGCGTTTGAAA
TACTTGGCCAGCAGACTAATGAAACCTCTTCTGCAGTTGATCGCTGAGGGTA
CCACCAAGCTCGCTTCCGTTCCCTCTGGCGGTGGTGGTGCTGCCGCCCC
CGCTGCTGGTGGCGCTGCCGCCGCTGAGGCCCCTGCTGCTGAGAAGGAGGA
GGAGAAGGAGGAGTCCGACGAGGACATGGGCTTCGGTCT
 **SEQ ID NO 50:**
> Published P2B sequences for C albicans gi/1 11229041|gb|AF317662.1|AF317662 Candida albicans 60S acidic ribosomal protein type P2-B (p2B) gene, complete cds 
GGAAAAAATGAAATACTTAGCTGCTTACTTATTGTTAGTTCAAGGTGGTAAC
ACCTCTCCATCAGCTTCTGATATCACCGCTTTATTGGAATCCGTTGGTGTTGA
AGCCGAAGAATCCAGATTACAAGCTTTATTGAAAGATTTGGAAGGTAAAGA
CTTGCAAGAATTGATTGCTGAAGGTAACACCAAATTAGCTTCTGTCCCATCC
GGTGGTGCTGCTGCTGGTGGTGCTTCTGCCTCTACTGGTGCCGCTGCTGGTG
GTGCTGCCGAAGCTGAAGAAGAAAAAGAAGAAGAAGCCAAAGAAGAATCT
GATGATGATATGGGTTTCGGTTTATTCGATTAGAGAAATTGCTTGTCGCCTT
TGCTGGTTTGAGAGAAGTATATTTCCATTATTTTGCATTATATATATATATAT
GTATTATAACTAATCTAATAAAAAAAATATGAAAACAAAAATGGCTTCTAT
ATGGCACTGTTTGCA
 **SEQ ID NO 51:**
> Published P2B sequences for C.glabrata gi|50284952|ref|XM_444905.1| Candida glabrata CBS138 hypothetical protein (CAGL0A03168g) partial mRNA 
ATGAAGTACTTGGCCGCTTACCTATTGTTGACCCAAGGTGGTAACGAATCTC
CAGCTGCTGCTGACATCAAGAAGGTTATCGAATCTGTTGGTATTGAAGCTGA
CGAAGCTAGAATCAACGAATTGTTGTCTGCTTTGGAAGGTAAGTCCTTGGAC
GAATTGATCGCTGAAGGTCAACAAAAGTTCGCCTCTGTTCCAGTTGGTGGTG
CTGCTGCTGGTGGTGCTTCCGCTGCTGCTGGTGGTGCCGCTGCCGGTGAAGC
TGCTGAAGAAAAGGAAGAAGAAGCTGCTGAAGAATCCGATGACGACATGG
GTTTCGGTTTGTTCGACTAA
 **SEQ ID NO 52:**
>gi|68465556|ref|7CM_718047.1| Candida albicans SC5314 cytosolic ribosomal acidic protein P2B (CaO19_5928) partial mRNA 
ATGAAATACTTAGCTGCTTACTTATTGTTAGTTCAAGGTGGTAACACCTCTC
CATCAGCTTCTGATATCACCGCTTTATTGGAATCCGTTGGTGTTGAAGCCGA
AGAATCCAGATTACAAGCTTTATTGAAAGATTTGGAAGGTAAAGACTTGCA
AGAATTGATTGCTGAAGGTAACACCAAATTAGCTTCTGTCCCATCCGGTGGT
GCTGCTGCTGGTGGTGCTTCTGCCTCTGCTGGTGCCGCTGCTGGTGGTGCTG
CTGAAGCTGAAGAAGAAAAAGAAGAAGAAGCCAAAGAAGAATCTGATGAT
GATATGGGTTTCGGTTTATTCGATTAG
 **SEQ ID NO 53:**
>gi|68465849|ref|XM_717900.1| Candida albicans SC5314 cytosolic ribosomal acidic protein P2B (Ca019_13349) partial mRNA 
ATGAAATACTTAGCTGCTTACTTATTGTTAGTTCAAGGTGGTAACACCTCTC
CATCAGCTTCTGATATCACCGCTTTATTGGAATCCGTTGGTGTTGAAGCCGA
AGAATCCAGATTACAAGCTTTATTGAAAGATTTGGAAGGTAAAGACTTGCA
AGAATTGATTGCTGAAGGTAACACCAAATTAGCTTCTGTCCCATCCGGTGGT
GCTGCTGCTGGTGGTGCTTCTGCCTCTGCTGGTGCCGCTGCTGGTGGTGCTG
CTGAAGCTGAAGAAGAAAAAGAAGAAGAAGCCAAAGAAGAATCTGATGAT
GATATGGGTTTCGGTTTATTCGATTAG
 **SEQ ID NO 54:**
> Published P2 sequence for A.fumigatus gi|71001323|ref|XM_750250.1| Aspergillus fumigatus Af293 60S acidic ribosomal protein P2/allergen Asp F 8 (AFUA_2G10100) mRNA, complete cds 
ATGAAGCACCTCGCCGCTTACCTCCTCCTCGCCCTTGCTGGCAACACCTCCC
CGTCCTCTGAGGATGTCAAGGCCGTCCTCTCTTCCGTTGGCATTGATGCCGA
TGAGGAGCGCCTGAACAAGCTCATTGCTGAGCTCGAGGGCAAGGACCTCCA
GGAGCTCATCGCCGAGGGTTCCACCAAGCTCGCTTCCGTTCCCTCCGGTGGT
GCTGCCGCCGCTGCTCCTGCCGCTGCCGGTGCCGCTGCCGGTGGTGCTGCTG
CTCCTGCCGCTGAGGAGAAGAAGGAGGAGGAGAAGGAGGAGTCCGACGAG
GACATGGGCTTCGGTCTTTTCGACTAA
 **SEQ ID NO 55:**
Published P2 sequence for N.fischeri gi|119480930|ref|XM_001260493.1| Neosartorya fischeri NRRL 181 60S acidic ribosomal protein P2/allergen Asp F 8 (NFIA_085510) mRNA, complete cds 
ATGAAGCACCTCGCCGCTTACCTCCTCCTCGCCCTTGCTGGCAACACCTCCC
CGTCCGCTGAGGATGTCAAGGCCGTCCTCTCTTCCGTTGGCATTGACGCCGA
TGAGGAGCGCCTGAACAAGCTCATTGCTGAGCTCGAGGGCAAGGACCTCCA
GGAGCTCATCGCCGAGGGTTCCACCAAGCTCGCTTCCGTTCCCTCCGGTGGT
GCTGCCGCCGCTGCTCCTGCCGCTGGCGGTGCCGCTGCCGGTGGTGCTGCTG
CTCCTGCCGCTGAGGAGAAGAAGGAGGAGGAGAAGGAGGAGTCCGACGAG
GACATGGGCTTCGGTCTCTTCGACTAA
 **SEQ ID NO 56:**
Published P2 sequence for A.clavatus gi|121715403|ref|Xm_001275310.1| Aspergillus clavatus NRRL 1 60S acidic ribosomal protein P2, putative (ACLA_069130) mRNA, complete cds 
ATGAAGCACCTCGCCGCTTACCTCCTCCTCGCCCTTGGTGGCAACGCCAGCC
CCTCCGCTGCTGATGTTAAGGAGGTTCTCTCTTCTGTCGGCATTGATGCTGAT
GAGGAGCGCCTCAACAAGCTCATTGCTGAGCTCGAGGGCAAGGACCTTCAG
GAGCTGATTGCTGAGGGTTCCACCAAGCTCGCTTCCATTCCCTCCGGCGGTG
CTGGTGGTGCTGCCCCCGCCGCTGGCGGTGCTGCCGCCGGTGGTGCTGCTGA
GGCCGCTCCCGCTGAGGAGAAGGAGGAGGAGAAGGAGGAGTCCGACGACG
ACATGGGCTTCGGTCTCTTCGACTAA
 **SEQ ID NO 57:**
Published P2 sequence for A.terreus gi|115395965|ref|XM_001213622.1| Aspergillus terreus NIH2624 predicted protein (ATEG_04444) mRNA, complete cds 
ATGAAGCACCTCGCCGCTTTCCTTCTCCTCGGCCTTGCCGGCAACACCTCCC
CCTCTGCTGAGGACATCAAGGCTGTCCTCTCCTCCGTCGGCATTGACGCTGA
TGAGGAGCGCCTCGGCCAGCTCCTGAAGGAGCTCGAGGGCAAGGACATCCA
GGAGCTCATCGCTCAGGGCTCTGAGAAGCTCGCCTCCGTTCCCTCTGGCGGT
GCTGCCGCTGGTGCTGCTGCTCCCGCCGCTGCCGCTGGCGGTGACGCTGCTG
CCCCCGCTGAGAAGAAGGAGGAGGAGAAGGAGGAGGAGTCCGACGAGGAC
ATGGGCTTCGGTCTCTTCGACTAA
 **SEQ ID NO 58:**
Published P2 sequence for A. niger gi|145252575|ref|XM_001397764.1| Aspergillus niger CBS 513.88 hypothetical protein (An16g04930) mRNA, complete cds 
ATGAAGTACCTCGCCGCCTACCTTCTGTTGGCCCTTGCTGGCAACAACACCC
CCTCCGCTGAGGACATCAAGTCCGTCCTCTCCGCCGTCGGCATTGACGCTGA
GGAGGAGCGCCTCCAGAAGCTCCTTGCTGAGCTTGAGGGCAAGGACCTCCA
GGAGCTCATCTCCGAGGGTACCCAGAAGCTCGCTTCCGTTCCCTCCGGTGGT
GCCGGTGCTGCTGCCGCTGCCCCCGCTGCCGGTGGCGCCGCTGCTGCTGAGG
CTCCCGCTGAGGAGAAGAAGGAGGAGGCTGCTGAGGAGTCCGATGAGGAC
ATGGGCTTCGGTCTCTTCGACTAA
 **SEQ ID NO 59:**
>gi|67539651|ref|XM_658508.1|Aspergillus nidulans FGSC A4 chromosomeI 
ATGAAGCACCTCGCAGCCTACCTCCTCCTCGCCCTTGCTGGCAACGAGTCTC
CCTCCGCCTCCGACATCAAGGAGGTTCTCTCTTCCGTCGGTGTTGACGCCGA
CGACGAGCGCCTCGAGAAGCTCATTGCTGAGCTCCAGGGCAAGGACATCAA
CGAGCTGATCGCTGAGGGTACCACCAAGCTTGCCTCCGTTCCCTCCGGCGGT
GCTGGTGGTGCTGCCCCTGCTGCCGCTGCCGGTGGTGCTGCTGCTGCCGAGG
 
CCCCCGCTGCTGAGAAGGAGGAGGAGAAGGAGGAGTCCGATGAGGACATG
GGCTTCGGTCTCTTCGACTAA
 SEQ ID NO 60:
>gi|11229039|gb|AF317661.1| Candida albicans 60S acidic ribosomal protein type P2-A (p2A) gene, complete cds 
GAATTCGATTATTGCATTCTGATATTCCCTGCTTTAAATGCATTTGGAAATAT
TTCGTATATCATGAGATATAATAACATTAATAGCATTTTCATGTTACTAACA
AGAATATAGTGAAATACTTAGCTGCTTACTTATTATTAGTTAACGCCGGTAA
CGCCACCCCATCTGCTGCCGATGTCAAAGCTGTTTTGTCAGCTGCTGATATT
GAAGTCGAAGAAGAAAAAGTTGAAAAATTGATCAGCGAATCGGACGGTAA
GAACGTCGAAGAATTGATTGCTGAAGGTAACGAAAAATTATCATCAGTCCC
ATCTGGTGCTCCAGCTGCTGCTGCTGGTGGTGCTTCTGCTGCCGCCGGTGGT
GAAGCCACTGAAGAAGCTGCTGAAGAAGAAGCTGCTGAAGAATCTGATGAC
GATATGAGTTTCGGTTTATTCGATTAAACGAGTCAACAGGCATCTCAAGATC
ACAGCATAAGG
 **A. fumigatus**
**SEQ ID NO 110:**
>A. FUM505.62 
TTACCTCCTCCTCGCCCTTGCTGGCAACACCTCCCCGTCCTCTGAGGATGTCA
AGGCCGTCCTCTCTTCCGTTGGCATTGATGCCGATGAGGAGCGCCTGAACAA
GCTCATTGCTGAGCTCGAGGGCAAGGACCTCCAGGAGGTTAGTAACTACAG
CTCGAAGATTACAGACTGGGAATTTTGGACTGGCGCTGACATCGAACTCTAC
AACAGCTCATTGCCGAGGGTTCCACCAAGCTCGCTTCCGTTCCCTCCGGTGG
TGCTGCCGCCGCTGCTCCTGCCGCTGCCGGTGCCGCTGCCGGTGGTGCTGCT
GCTCCTGCCGCTGAGGAGAAGAAGGAGGAGGAGAAGGAGGAGTCCGACGA
GGACATGGGCTTCGGTCT
 **A. flavus**
**SEQ ID NO 61:**
>A.flavus2199\(P2) 
TCGCCCTCGCTGGCAACTCCACCCCCTCCGTTGAGGACATCAAGAGCGTTCT
CTCTTCCGTCGGTATTGATGCCGATGAGGAGCGCCTCCAGAAGGTCATCTCC
GAGCTCGAGGGCAAGGACCTCCAGCAGGTTCGTAATACGTTGAATGATTGA
GACATAGGGCGCCTGCTGACCATCTACTCACTACAGCTGATCACTGAGGGT
AGCGAGAAGCTCGCTACCGTTCCCTCCGGTGGTGCTGGTGCCGCTGCCCCTG
CTGCTGGCGGTGCCGCTGCCGGTGGTGACGCCCCCGCCGCTGAGGAGAAGG
AGGAAGAGAAGGAGGAGTCCGATGAGGACATGGGCTTCGGTCT
 **A. niger**
**SEQ ID NO 62:**
>A. nig2828\(P2F) 
CTACCTTCTGTTGGCCCTTGCTGGCAACAACACCCCCTCCGCTGAGGACATC
AAGTCCGTCCTCTCCGCCGTCGGCATTGACGCTGAGGAGGAGCGCCTCCAG
AAGCTCCTTGCTGAGCTTGAGGGCAAGGACCTCCAGGAGGTCAGTTAACGC
CCTTAAAAATCTACCAAGAAATTTTGCGATTACAAGATGGAATACTGACAA
TGGTTTTTCTACAACAGCTCATCTCCGAGGGTACCCAGAAGCTCGCTTCCGT
TCCCTCCGGTGGTGCCGGTGCTGCTGCCGCTGCCCCCGCTGCCGGTGGCGCC
 
GCTGCTGCTGAGGCTCCCGCTGAGGAGAAGAAGGAGGAGGCTGCTGAGGA
GTCCGATGAGGACATGGGCTTCGGTCT
 **A. terreus**
**SEQ ID NO 63:**
>A. terr307 
TTTCCTTCTCCTCGGCCTTGCCGGCAACACCTCCCCCTCTGCTGAGGACATCA
AGGCTGTCCTCTCCTCCGTCGGCATTGACGCTGATGAGGAGCGCCTCGGCCA
GCTCCTGAAGGAGCTCGAGGGCAAGGACATCCAGGAGGTTAGTGATCACCA
TAACTTCGGACTACTGCGATGAAAACGCGCCATACTAACGGATTATACAGC
TCATCGCTCAGGGCTCTGAGAAGCTCGCCTCCGTTCCCTCTGGCGGTGCTGC
CGCTGGTGCTGCTGCTCCCGCCGCTGCCGCTGGCGGTGACGCTGCTGCCCCC
GCTGAGAAGAAGGAGGAGGAGAAGGAGGAGGAGTCCGACGAGGACATGG
GCTTCGGTCT
 **SEQ ID NO 64:**
>A. terr5677 
TTTCCTTCTCCTCGGCCTTGCCGGCAACACCTCCCCCTCTGCTGAGGACATCA
AGGCTGTCCTCTCCTCCGTCGGCATTGACGCTGATGAGGAGCGCCTCGGCCA
GCTCCTGAAGGAGCTCGAGGGCAAGGACATCCAGGAGGTTAGTGATCACCA
TAACTTCGGACTACTGCGATGAAAACGCGCCATACTAACGGATTATACAGG
TCATCGCTCAGGGCTCTGAGAAGCTCGCCTCCGTTCCCTCTGGCGGTGCTGC
CGCTGGTGCTGCTGCTCCCGCCGCTGCCGCTGGCGGTGACGCTGCTGCCCCC
GCTGAGAAGAAGGAGGAGGAGAAGGAGGAGGAGTCCGACGAGGACATGG
GCTTCGGTCT
 **SEQ ID NO 65:**
>A. terr2729 
TTTCCTTCTCCTCGGCCTTGCCGGCAACACCTCCCCCTCTGCTGAGGACATCA
AGGCTGTCCTCTCCTCCGTCGGCATTGACGCTGATGAGGAGCGCCTCGGCCA
GCTCCTGAAGGAGCTCGAGGGCAAGGACATCCAGGAGGTTAGTGATCACCA
TAACTTCGGACTACTGCGATGAAAACGCGCCATACTAACGGATTATACAGC
TCATCGCTCAGGGCTCTGAGAAGCTCGCCTCCGTTCCCTCTGGCGGTGCTGC
CGCTGGTGCTGCTGCTCCCGCCGCTGCCGCTGGCGGTGACGCTGCTGCCCCC
GCTGAGAAGAAGGAGGAGGAGAAGGAGGAGGAGTCCGACGAGGACATGG
GCTTCGGTCT
 **SEQ ID NO 66:**
>A. terr601-65 
TTTCCTTCTCCTCGGCCTTGCCGGCAACACCTCCCCCTCTGCTGAGGACATCA
AGGCTGTCCTCTCCTCCGTCGGCATTGACGCTGATGAGGAGCGCCTCGGCCA
GCTCCTGAAGGAGCTCGAGGGCAAGGACATCCAGGAGGTTAGTGATCACCA
TAACTTCGGACTACTGCGATGAAAACGCGCCATACTAACGGATTATACAGC
TCATCGCTCAGGGCTCTGAGAAGCTCGCCTCCGTTCCCTCTGGCGGTGCTGC
CGCTGGTGCTGCTGCTCCCGCCGCTGCCGCTGGCGGTGACGCTGCTGCCCCC
GCTGAGAAGAAGGAGGAGGAGAAGGAGGAGGAGTCCGACGAGGACATGG
GCTTCGGTCT
 **A. candidus**
**SEQ ID NO 67:**
>A. Cand 225-8\(P2F) 
TGCTCCTCGGCCTCGCMGGCAACGAGACTCCCTCCGCTGCCGACATCAAGG
GCGTTCTGTCCGCCGTCGGCATTGACGCCGATGAGGACCGTCTCTCCAAGCT
CCTCTCCGAGCTTGAGGGCAAGGACATCAACGAGGTTCGTATCTCACAGGA
ATCGCACACGTAACAGAGTCAACAAATACTAATCCCCCGTGCAGCTGATCG
CCCAGGGCTCCGAGAAGCTTGCTTCCGTTCCCTCCGGTGGTGCCGCTGGTGG
TGCCGCTGCCGCCCCTGCCGCCGCCGCTGGTGGTGACGCTCCCGCCCAGGAG
AAGGAGGAGGAGAAGGAGGAGTCCGATGAGGACATGGGCTTCGGTCT
 **SEQ ID NO 68:**
>A. Cand 14607\(P2F) 
TGCTCCTCGGCCTCGCCGGCAACGAGTCTCCCTCCGCTGCCGACATCAAGGG
CGTTCTGTCCGCCGTCGGCATTGACGCCGATGAGGAGCGTCTCTCCAAGCTC
CTCTCCGAGCTTGAGGGCAAGGACATCAACGAGGTTCGTATCCTGGAATCG
CACCGAGACAGAGTCAACAAATACTAATCCCCCGTGCAGCTGATCGCCCAG
GGTACCGAGAAGCTTGCTTCCGTTCCCTCCGGTGGCGCCGGCGCTGCTGCTG
CCGCCCCTGCCGCCGCTGCTGGTGGTGAGGCCGCCGCTGAGGAGAAGAAGG
AGGAGGAGAAGGAGGAGTCCGATGAGGACATGGGCTTCGGTCT
 **SEQ ID NO 69:**
>A. Cand-9695\(APF) 
GGCAACCGAGTCTCCCTCCGCTGCCGACATCAAGGGCGTTCTGTCCGCCGTC
GGCATTGACGCCGATGAGGAGCGTCTCTCCAAGCTCCTCTCCGAGCTTGAGG
GCAAGGACATCAACGAGGTTCGTATCCTGGAATCGCACCGAGACAGAGTCA
ACAAATACTAATCCCCCGTGCAGCTGATCGCCCAGGGTACCGAGAAGCTTG
CTTCCGTTCCCTCCGGTGGCGCCGGCGCTGCTGCTGCCGCCCCTGCCGCCGC
TGCTGGTGGTGAGGCCGCCGCTGAGGAGAAGAAGGAGGAGGAGAAGGAGG
AGTCCGATGAGGACATGGGCTTCGGTCT
 **A. clavatus**
**SEQ ID NO 70:**
>A. clav7944\(P2F) 
TTACCTCCTCCTCGCCCTTGGTGGCAACGCCAGCCCCTCCGCTGCTGATGTT
AAGGAGGTTCTCTCTTCCGTCGGCATTGATGCTGATGAGGAGCGCCTCAACA
AGCTCATTGCTGAGCTCGAGGGCAAGGACCTTCAGGAGGTTAGTTTACGCT
GGTCTACGAGAGGAAGATTGTGACAAGATGCTAACGGAAAATTTCTTCAAC
AGCTGATTGCTGAGGGTTCCACCAAGCTCGCTTCCATTCCCTCCGGCGGTGC
TGGTGGTGCTGCCCCCGCCGCTGGCGGTGCTGCCGCCGGTGGTGCTGCTGAG
GCCGCTCCCGCTGAGGAGAAGGAGGAGGAGAAGGAGGAGTCCGACGACGA
CATGGGCTTCGGTCT
 **SEQ ID NO 71:**
>A. clav443\(P2F) 
TTACCTCCTCCTCGCCCTTGGTGGCAACGCCAGCCCCTCCGCTGCTGATGTT
AAGGAGGTTCTCTCTTCCGTTGGCATTGATGCTGATGAGGAGCGCCTCAATA
 
AGCTCATTGCTGAGCTTGAGGGCAAGGACCTTCAGGAGGTTAGTTTACGCC
AATCCGCGATATGAAAATTGCGACATGATGCTAACAGGAGAATTTTTCAAC
AGCTGATTGCTGAGGGTTCCACCAAGCTCGCTTCCATTCCTTCCGGCGGTGC
TGCCGGTGCTGCCCCTGCCGCTGGCGCTGCTGCCGGTGGTGCCGCTGAGGCC
GCTCCCGCTGAGGAGAAGGAGGAGGAGAAGGAGGAGTCCGACGACGACAT
GGGCTTCGGTCT
 **A. glaucus**
**SEQ ID NO 72:**
>A. glau2425\(P2F) 
ACACGGTGGACTGGCTGGCCGGAAGAGCCGTTGCTGGTGTAAGTTTTGGTA
GGACCGTTCAGCTCCRGAKGTCTTTGGGGACAATGGCGACGAGGGCTGTCG
ATCGTTAGTGTACATTCCAAGGAAGGTAAAAGAAAAAGAATGCGACATACA
GTAAGTTGAGCCACTCTGACGCTGGCAGTCATCGCAGTGGTCATAAGCTGTA
GCGAGCGGCTGCTCGATGTTGACCGAGTAGGTGACGGCTTTGCAGAGACAG
TGACCGGAAATAGGCATTTTGGACGAGAGGCAGAATATAATTGGACTATAG
ACTGACAATGATGATGACGATACTGAGGACCTTTGGGAGGAATGAAACAGA
TATTTATACCCTCTCCGACCTCGTGTAGTCCATCTGTCTTTCGCTGTCTCTCG
GTGGACATGGGCTTCGGTCT
 **SEQ ID NO 73:**
>A. glau542 
GCGTTGGTGCACGACGGTACCAAGGGGACTCTCATACTCCTTAATTCTCTGG
CCGGTCCAGTGTCGCGACTCRTATGGAGCACATTAGCAGATATCCCACAGA
GCTACCATGTCGTCCAGGCTCTTTGTCTGCTTTGCACATGGCCTTTTCCAACC
AGCAGTACCTCCACAGACCCGACGTTCATGCTGTGTGGTATGATGATGCAA
GTCGCCATGCAACTTGGTCTTCACCGGCCTTCGCACACTCAGGACTTTASCA
AGTTCACAGTGGAGCTGATTGAGGAGGAGCTCAGGGATAAAGTGAGGACAT
GGGCTTCGGTCT
 **SEQ ID NO 74:**
>A.glaucus29771\(P2) 
GCGTTGGAGCACGACTGTACCAGGATACTCATCATCTCCTTAATTTCTGGC
CGGTCCAGCGYCGCGACTAGTATGGAGCACATTATCAGACATCCGACAGAG
CTACCATGTCGTAAAGGCTCTTTGTCTGCTCTGCACATGGCCTTTTCCGACCA
GCAGTACCTCCACAGACCCTACKTTTATGCTGTGTGGTATGATGATGCAAGT
CGCCATGCAGCTTGGTCTTCACCGGCCTTCGCACACTCAGGACTTTAGCAAG
TTTACAGTGGAGCTGATTGAGGAGGAACTAAGGGACAAAGTGAGGACATGG
GCTTCGGTCT
 **A. versicolor**
**SEQ ID NO 75:**
>A.Vers6898 
CGCCCTTGCTGGCAACCAGAGCCCCTCTGCTTCCGATGTTAAGGAGGTTCTC
TCCTCCGTCGGTGTTGACGCTGACTCTGAGCGCCTCGAGAAGCTCATCGCTG
AGCTCCAGGGCAAGGACATCAACGAGGTTCGTTTTGACAGAACCGCTTGAA
ATTCTTGGCAGCAGACTAATGAAACATTTTCTGCAGTTGATCGCTGAGGGTA
CCACTAAGCTCGCTTCCGTTCCCTCTGGCGGTGCTGGTGCTGCTGCTCCC
 
GCTGCTGGTGGCGCTGCTGCCGCTGAGGCCCCTGCCGCCGAGAAGGAGGAG
GAGAAGGAGGAGTCCGACGAGGACATGGGCTTCGGTCT
 **SEQ ID NO 76:**
>lus66\(P2BF) sequence generated for *C. lusitaniae* 
KTATTGTTGGKCAACGCTGGTAACACCGCCCCATCTGCTGCTGACGTCAAGA
AGGTCTTGGAATCCGTCTCTATTGAGGTTGAGGACGACAAGGTTGAGAAGT
TGTTGGCTGAAGTTGAAGGCAAGAACGTCGAAGAGTTGATTGCCGAGGGTA
ACGAGAAGTTGTCTTCTGTTCCATCTGGTGCTCCAGCTGCTGCTGGTGCCGC
TGCTGCTTCTGGTTCTACTGAGGCTGCTGCTGAAGAGCCACAAGAAGAAGA
GAAGGAGGAGTCTGACGACGACATGGGTTTCGGTTTATTCGATTA
 **SEQ ID NO 77:**
>fam1\(P2BF) sequence generated for *C*.*famata* 
CTTCTCCATCCGCTTCTGACATCAGTAGTTTATTAGAAACCGTTGGTGCTGA
AGCTGACGAAGCTAGAATCAGTGCTTTATTGAAGGACTTAGAAGGTAAGCA
AGTCGCTGACTTAATTGCTGAAGGTCAAACCAAGTTGGCTTCCGTTCCAACT
GGTGGTGCTGGTGCTGCTGCTGGTGGTGCCGCTGCTGCTTCTGGTGATGCCG
GTGCAGCTGCTGCTGAAGAAGAAAAGGAAGAAGAAAAGGAAGAATCCGAC
GATGACATGGGTTTCGGTTTATTCGATTA
 **SEQ ID NO 78:**
>fam2\(P2BF) sequence generated for *C. famata* 
CTTCTCCATCAGCCTCTGACGTCAGTGCTTTATTAGAAACCGTTGGTGCTGA
AGTTGACCAAGGTAGAGTTAGTGCTTTATTGAAGGACTTAGAAGGTAAGCA
AGTTGCCGACTTAATTGCTGAAGGTCAAACCAAGTTAGCTTCTGTCCCAACC
GGTGGTGCTGCTTCTGCTGGTGGTGCTGCCGCTGCTTCTGGTGCTGCCGGTG
CAGCTGCTGTTGAAGAAGAAAAGGAAGAAGAAAAGGAAGAATCCGATGAA
GATATGGGTTTCGGTTTATTCGATT
 **SEQ ID NO 79:**
>fam5\(P2BF) sequence generated for *C. famata* 
CTTCTCCATCAGCCTCTGACGTCAGTGCTTTATTAGAAACCGTTGGTGCTGA
AGTTGACCAAGGTAGAGTCAGTGCTTTATTGAAGGACTTAGAAGGTAAGCA
AGTCGCCGACTTAATTGCTGAAGGTCAAACCAAGTTAGCTTCTGTCCCAACT
GGTGGTGCTGCTTCTGCTGGTGGTGCTTCCGCTGCTGCTTCTGGTGATGCCG
GTGCAGCTGCTGCTGAAGAAGAAAAGGAAGAAGAAAAGGAAGAATCCGAT
GAAGATATGGGTTTCGGTTTATTCGATTA
 **SEQ ID NO 80:**
>haem54\(P2BF) sequence generated for *C. haemuloni* 
TTTCGGTTTATTCGATTAACACTTCCCCAGCTGCCTCTGACATCAAGAAGGT
GTTGGAGTCTGTCTCCATCGAGGTTGAGGACGACAAGGTCGAGAAGTTGTT
GGCTGAGGTCGAGGGCAAGAACGCCGAGGAGTTGATTGCCGAGGGTAACG
AGAAGTTGTCTTCTGTCCCAACTGGTGCTCCAGCTGGTGGTGCTGCCGCTGC
TGGTGGTGCTGCTCCAGAGGCTGCTGCTGAGAAGGAAGAGGAGGCCGCTGC
CGAGGAGTCTGACGACGACATGGGTTTCGGTTTATTCGATTA
 **SEQ ID NO 81:**
>hacm55\(P2BF) sequence generated for *C. haemuloni* 
TTATTGTTGGTCAACGCCGGTAACACTTCCCCAGCTGCCTCTGACATCAAGA
AGGTGTTGGAGTCTGTCTCCATCGAGGTTGAGGACGACAAGGTCGAGAAGT
TGTTGGCTGAGGTCGAGGGCAAGAACGCCGAGGAGTTGATTGCCGAGGGTA
ACGAGAAGTTGTCTTCTGTCCCAACTGGTGCTCCAGCTGGTGGTGCTGCCGC
TGCTGGTGGTGCTGCTCCAGAGGCTGCTGCTGAGAAGGAAGAGGAGGCCGC
TGCCGAGGAGTCTGACGACGACATGGGTTTCGGTTTATTCGATTA
 **SEQ ID NO 82:**
>pul36\(P2BF) sequence generated for *C. pulcherrima* 
ACACCTCTCCATCCGCCGCCGATGTCAAGAAGGTCTTGGAGTCCGTTTCCAT
CGAGGTTGAGGAGGACAAGGTCGAGAAGTTGCTCGCTGAGGTCGAGGGCA
AGAGCGTCGAGGACTTGATCGCTGAGGGTAACGAGAAGTTGTCTTCTGTCC
CAACTGGTGGCCCAGCCGCCGGTGGTGCCGCTGCCGCTGCTGGTGGTGACG
CCGCTCCTGCCGAGGAGGCCGCTGAGGAGGCCGCCGAGGAGTCTGACGACG
ACATGGGTTTCGGTTTATTCGATTA
 **SEQ ID NO 83:**
>pul39\(P2BF) sequence generated for *C*. *pulcherrima* 
CATCCGCCGCCGATGTCAAGRAGGTCTTGGAGTCCGTTTCCATCGAGGTTGA
GGAGGACAAGATCGAGAAGTTGCTCGCTGAGGTCGAGGGCAAGAGCGTCG
AGGACTTGATCGCTGAGGGTAACGAGAAGTTGTCTTCTGTCCCAACTGGTGG
CCCAGCCGCCGGTGGTGCCGCTGCCGCTGCCGGTGGTGACGCCGCTCCTGCC
GAGGAGGCCGCTGAGGAGGCCGCCGAGGAGTCTGACGACGACATGGGTTTC
GGTTTATTCGATTA
 **SEQ ID NO 84:**
>U50\(P2BF) sequence generated for *C. utilis* 
ACCGCCGACAAGATCACCTCCGTCTTGGAGTCTGTCGGTATTGAGGTTGAGG
AGTCCCAAGTCACCGAGTTGATCTCTGCCCTTGAGGGTAAGTCCGTTGAGGA
GCTCATTGCTGAAGGTAACGAGAAGTTGGCTTCTGTTCCAACCGGTGGTGCT
GGTGCTGCTCCAGCTGCCGGTGCCGGTGCTGCTGATGCTGATGCTCCAGCTG
AGGCTGCTGAGGAGGCTGCTGAGGAGGAGTCTGACGATGACATGGGTTTCG
GTTTATTCGATTA
 **Seq ID No 85 P2ForA**
**GAGGAGCGCCT**
**Seq ID No 86 P2ForB**
GAGGAGCGCCTC
**Seq ID No 87 P2ForC**
GGAGGAGCGCCTC
**Seq ID No 88 P2ForD**
TGAGGAGCGCCTC
**Seq ID No 89 P2RevA**
CCGGAGGGAACGGA
**Seq ID No 90 P2RevB**
CCGGAGGGAACGG
**SeqID No 91 P2FumP**
ACTACAGCTCGAAGATTA
**Seq ID No 92 P2FlavP**
ACGTTGAATGATTGAGAC
**Seq ID No 93 P2NigP**
TTGCGATTACAAGATGGAA
**Seq ID No 94 P2TerrP**
CTTCGGACTACTGCGATGA
**Seq ID No 95 CAP2BF (For )**
ACCTCTCCATCAGCTTCTG
**Seq ID No 96 CGP2BF (For )**
ACCTCTGTCTTATCATCTGTCG
**Seq ID No 97 CGP2BF (For )**
AAGAAGGTTATCGAATCTGTTG
**Seq ID No 98 CTP2BF (For )**
TCCGCTTTATTGGAACAAGTTG
**Seq ID No 99 CPP2BF (For )**
TCCTCATTGTTGGAATCCGTTG
**Seq ID 100 CAP2BR (Rev )**
TCAGCAATCAATTCTTGC
**SEQ ID 101 CKP2BR (Rev )**
CTCTTCGACGGACTTACC
**SEQ ID 102 CGP2BR (Rev )**
TTCGTCCAAGGACTTACC
**SEQ ID 103 CTP2BR (Rev )**
TTCTTGCAAGTCTTTACC
**SEQ ID 104 CPP2BR (Rev )**
CTCGTTGATGTCTTTACC
**SEQ ID 105 CAP2BP (P)**
ACCGCTTTATTGGAATCCGTTG
**SEQ ID 106 CKP2BP (P)**
ATCCGACAAGTTAGACAAGTTAATC
**SEQ ID 107 CGP2BP (P)**
AGAATCAACGAATTGTTGTCTGC
**SEQ ID 108 CTP2BP (P)**
ATCTTCCAAATTAGACTTATTGTTGA
**SEQ ID 109 CPP2BP (P)**
GAAGAATCAAGATTATCTACCTTGTTG

### References:

Abramczyk D, Tchórzewski M, Krokowski D, Boguszewska A, Grankowski N. Overexpression, purification and characterization of the acidic ribosomal P-proteins from Candida albicans. Biochim Biophys Acta. 2004 Jun 11;1672(3):214-23.
Bailey-Serres J, Vangala S, Szick K, and Lee CH. Acidic phosphoprotein complex of the 60S ribosomal subunit of maize seedling roots. Components and changes in response to flooding. Plant Physiol. 1997 August; 114(4): 1293-1305.
Newton CH, Shimmin LC, Yee J, Dennis PP. (1990) A family of genes encode the multiple forms of the Saccharomyces cerevisiae ribosomal proteins equivalent to the Escherichia coli L12 protein and a single form of the L10-equivalent ribosomal protein. JBacteriol. 1990; 172: 579-588
Tchórzewski M, Krokowski D, Boguszewska A, Liljas A, Grankowski N. Structural characterization of yeast acidic ribosomal P proteins forming the P1A-P2B heterocomplex. Biochemistry. 2003 Apr 1;42(12):3399-408.
Tchórzewski M, Boguszewska A, Dukowski P, Grankowski N. Oligomerization properties of the acidic ribosomal P-proteins from Saccharomyces cerevisiae: effect of P1A protein phosphorylation on the formation of the P1A-P2B hetero-complex. Biochim Biophys Acta. 2000 Dec 11;1499(1-2):63-73.
Wool IG, Chan YL, Gluck A, Suzuki K The primary structure of rat ribosomal proteins PO, P1 and P2 and a proposal for a uniform nomenclature for mammalian and yeast ribosomal proteins. Biochimie 1991; 73: 861-870.

## Claims

1. A diagnostic kit for the detection and discrimination of Candida species from other yeast and/or fungal species, comprising an oligonucleotide probe capable of binding to at least a portion of the P2, P2A or P2B genes or its corresponding mRNA, wherein the probe is selected from the group consisting of SEQ ID NO 9, and sequences of between 10 and 100 nucleotides in length which preferentially hybridize to the nucleotide sequence SEQ ID NO 9, and which are able to discriminate Candida species from other yeast and/or fungal species and sequences of between 10 and 100 nucleotides in length which are complementary thereto.

2. A kit as claimed in claim 1 comprising a primer for amplification of at least a portion of the P2, P2A and/or P2B gene, the primer being a forward or reverse primer for a portion of the P2, P2A and/or P2B gene.

3. A kit as claimed in any preceding claim comprising at least one forward *in vitro* amplification primer and at least one reverse *in vitro* amplification primer, the forward amplification primer being selected from the group consisting of SEQ ID NO 1, sequences of between 10 and 100 nucleotides in length which are complimentary to sequences that preferentially hybridize to the nucleotide sequence SEQ ID NO 1, and are able to discriminate Candida species from other yeast and/or fungal species, and can also act as a forward amplification primer and the reverse amplification primer being selected from the group consisting of SEQ ID NO 2, sequences of between 10 and 100 nucleotides in length which are complementary to sequences that preferentially hybridize to the nucleotide sequence SEQ ID NO 2, and which are able to discriminate Candida species from other yeast and/or fungal species, and can also act as a reverse amplification primer.

4. A nucleic acid molecule selected from the group consisting of: SEQ ID NO 1, 2, 9, and sequences of between 10 and 100 nucleotides in length which preferentially hybridize to the nucleotide sequence SEQ ID NO 1, 2, 9 and which are capable of binding to at least a portion of the P2, P2A or P2B genes or its corresponding mRNA, and which are able to discriminate Candida species from other yeast and/or fungal species, and sequences of between 10 and 100 nucleotides in length which are complementary thereto and which are able to discriminate Candida species from other yeast and/or fungal species.

5. A method of discriminating a target yeast or fungal species from other yeast or fungal species in a test sample comprising the steps of:
(i) Mixing the test sample with at least one oligonucleotide probe capable of binding to at least a portion of the P2, P2A and/or P2B gene of the target species or its corresponding mRNA under appropriate conditions;
(ii) hybridizing under a high stringency conditions any nucleic acid that may be present in the test sample with the oligonucleotide to form a probe:target duplex; and
(iii) determining whether a probe:target duplex is present; the presence of the duplex positively identifying the presence of the target organism in the test sample, wherein the probe is selected from the group consisting of SEQ ID NO 9, sequences of between 10 and 100 nucleotides in length which preferentially hybridize to the nucleotide sequence SEQ ID NO 9 and are able to discriminate Candida species from other yeast and/or fungal species and sequences of between 10 and 100 nucleotides in length complementary thereto also capable of acting as a probe for the P2B gene.

6. Use of a kit as claimed in any one of claims 1 to 3 or a nucleic acid molecule as claimed in claim 4 in a diagnostic assay to measure the titre of a target yeast or fungal species in a patient; in a diagnostic assay to detect the presence of a target yeast or fungal species; or in a diagnostic assay to measure a target yeast or fungal contamination in an environment..

7. A method of assessing the efficacy of a treatment regime designed to reduce yeast and/or fungal titre in a patient comprising use of a kit as claimed in any one of claims 1 to 3 or a nucleic acid molecule as claimed in claim 4 at one or more key stages of the treatment regime, wherein the key stages comprise before treatment, during treatment and after treatment.

8. Use as claimed in claim 6, wherein the environment is a hospital, a food sample, an environmental sample e.g. water, an industrial sample such as an in-process sample or an end product requiring bioburden or quality assessment.

## Patentansprüche

1. Diagnose-Kit zum Nachweis und zur Unterscheidung von Candida-Arten von anderen Hefe- und/oder Pilzarten, umfassend eine Oligonucleotid-Sonde, die in der Lage ist, an mindestens einen Abschnitt der P2-, P2A- oder P2B-Gene oder deren entsprechender mRNA zu binden, wobei die Sonde ausgewählt ist aus der Gruppe bestehend aus SEQ ID NO:9 und Sequenzen mit einer Länge zwischen 10 und 100 Nucleotiden, die vorzugsweise an die Nucleotidsequenz SEQ ID NO:9 hybridisieren und die in der Lage sind, Candida-Arten von anderen Hefe- und/oder Pilzarten zu unterscheiden, und Sequenzen mit einer Länge zwischen 10 und 100 Nucleotiden, die dazu komplementär sind.

2. Kit wie in Anspruch 1 beansprucht, umfassend einen Primer zur Amplifikation von mindestens einem Abschnitt des P2-, P2A- und/oder P2B-Gens, wobei der Primer ein Vorwärts- oder Rückwärtsprimer für einen Abschnitt des P2-, P2A- und/oder P2B-Gens ist.

3. Kit wie in einem der vorherigen Ansprüche beansprucht, umfassend mindestens einen *in vitro*-Vorwärtsamplifikationsprimer und mindestens einen *in vitro*-Rückwärtsamplifikationsprimer, wobei der Vorwärtsamplifikationsprimer ausgewählt ist aus der Gruppe bestehend aus SEQ ID NO:1, Sequenzen mit einer Länge zwischen 10 und 100 Nucleotiden, die komplementär sind zu Sequenzen, die vorzugsweise an die Nucleotidsequenz SEQ ID NO:1 hybridisieren und die in der Lage sind, Candida-Arten von anderen Hefe- und/oder Pilzarten zu unterscheiden und die auch als ein Vorwärtsamplifikationsprimer fungieren können, und wobei der Rückwärtsamplifikationsprimer ausgewählt ist aus der Gruppe bestehend aus SEQ ID NO:2, Sequenzen mit einer Länge zwischen 10 und 100 Nucleotiden, die komplementär sind zu Sequenzen, die vorzugsweise an die Nucleotidsequenz SEQ ID NO:2 hybridisieren und die in der Lage sind, Candida-Arten von anderen Hefe- und/oder Pilzarten zu unterscheiden und die auch als ein Rückwärtsamplifikationsprimer fungieren können.

4. Nucleinsäuremolekül, das ausgewählt ist aus der Gruppe bestehend aus SEQ ID NO:1, 2, 9 und Sequenzen mit einer Länge zwischen 10 und 100 Nucleotiden, die vorzugsweise an die Nucleotidsequenz SEQ ID NO:1, 2, 9 hybridisieren und die in der Lage sind, an mindestens einen Abschnitt der P2-, P2A- oder P2B-Gene oder deren entsprechender mRNA zu binden und die in der Lage sind, Candida-Arten von anderen Hefe- und/oder Pilzarten zu unterscheiden, und Sequenzen mit einer Länge zwischen 10 und 100 Nucleotiden, die komplementär dazu sind und die in der Lage sind, Candida-Arten von anderen Hefe- und/oder Pilzarten zu unterscheiden.

5. Verfahren zur Unterscheidung einer Ziel-Hefeart oder Ziel-Pilzart von anderen Hefe- oder Pilzarten in einer Testprobe, umfassend die Schritte:
(i) Mischen der Testprobe mit mindestens einer Oligonucleotid-Sonde, die unter geeigneten Bedingungen in der Lage ist, an mindestens einen Abschnitt des P2-, P2A- und/oder P2B-Gens der Ziel-Art oder dessen entsprechender mRNA zu binden;
(ii) Hybridisieren einer beliebigen Nucleinsäure, die in der Testprobe enthalten sein könnte, unter hoch stringenten Bedingungen mit dem Oligonucleotid, um einen Sonde-Ziel Duplex zu bilden; und
(iii) Bestimmen ob ein Sonde-Ziel Duplex vorhanden ist; wobei das Vorhandensein des Duplex das Vorhandensein des Ziel-Organismus in der Testprobe als positiv identifiziert, wobei die Sonde ausgewählt ist aus der Gruppe bestehend aus SEQ ID NO:9, Sequenzen mit einer Länge zwischen 10 und 100 Nucleotiden, die vorzugsweise an die Nucleotidsequenz SEQ ID NO:9 hybridisieren, und die in der Lage sind, Candida-Arten von anderen Hefe- und/oder Pilzarten zu unterscheiden, und dazu komplementären Sequenzen mit einer Länge zwischen 10 und 100 Nucleotiden, die auch in der Lage sind, als Sonde für das P2B-Gen zu fungieren.

6. Verwendung eines wie in einem der Ansprüche 1 bis 3 beanspruchten Kits oder eines wie in Anspruch 4 beanspruchten Nucleinsäuremoleküls in einem Diagnose-Assay zur Messung des Titers einer Ziel-Hefe oder von Ziel-Pilzarten in einem Patienten; in einem Diagnose-Assay zum Nachweis des Vorhandenseins einer Ziel-Hefe oder von Ziel-Pilzarten; oder in einem Diagnose-Assay zur Messung einer Kontamination mit einer Ziel-Hefe oder Ziel-Pilzart in einer Umwelt.

7. Verfahren zum Beurteilen der Wirksamkeit eines Behandlungsplans, der dafür ausgelegt ist, Hefe- und/oder Pilztiter in einem Patienten zu reduzieren, umfassend die Verwendung eines wie in einem der Ansprüche 1 bis 3 beanspruchten Kits oder eines wie in Anspruch 4 beanspruchten Nucleinsäuremoleküls in einem oder mehreren Hauptabschnitt(en) des Behandlungsplans, wobei die Hauptabschnitte vor Behandlung, während der Behandlung und nach der Behandlung umfassen.

8. Verwendung wie in Anspruch 6 beansprucht, wobei die Umwelt ein Krankenhaus, eine Nahrungsmittelprobe, eine Umweltprobe, z.B. Wasser, eine industrielle Probe, wie eine In-Prozess-Probe, oder ein Endprodukt, das eine Keimbelastungs- oder Qualitätsanalyse erfordert, ist.

## Revendications

1. Trousse de diagnostic pour la détection et la distinction des espèces Candida des autres espèces de levure et/ou fongiques, comprenant une sonde oligonucléotidique capable de se lier à au moins une portion du gène P2, P2A ou P2B ou son ARNm correspondant, où la sonde est choisie dans le groupe constitué par SEQ ID NO 9, et des séquences d'une longueur comprise entre 10 et 100 nucléotides, qui s'hybrident préférentiellement à la séquence nucléotidique SEQ ID NO 9, et qui sont capables de distinguer les espèces Candida des autres espèces de levure et/ou fongiques, et des séquences d'une longueur comprise entre 10 et 100 nucléotides qui leur sont complémentaires.

2. Trousse telle que revendiquée dans la revendication 1, comprenant une amorce pour l'amplification d'au moins une portion des gènes P2, P2A et/ou P2B, l'amorce étant une amorce directe ou une amorce inverse pour une portion des gènes P2, P2A et/ou P2B.

3. Trousse telle que revendiquée dans l'une quelconque des revendications précédentes, comprenant au moins une amorce d'amplification directe in vitro et au moins une amorce d'amplification inverse in vitro, l'amorce d'amplification directe étant choisie dans le groupe constitué par SEQ ID NO 1, des séquences d'une longueur comprise entre 10 et 100 nucléotides, qui sont complémentaires aux séquences qui s'hybrident préférentiellement à la séquence nucléotidique SEQ ID NO 1, et sont capables de distinguer les espèces Candida des autres espèces de levure et/ou fongiques, et peuvent également servir d'amorce d'amplification directe, et l'amorce d'amplification inverse étant choisie dans le groupe constitué par SEQ ID NO 2, des séquences d'une longueur comprise entre 10 et 100 nucléotides, qui sont complémentaires aux séquences qui s'hybrident préférentiellement à la séquence nucléotidique SEQ ID NO 2, et sont capables de distinguer les espèces Candida des autres espèces de levure et/ou fongiques, et peuvent également servir d'amorce d'amplification inverse.

4. Molécule d'acide nucléique choisie dans le groupe constitué par : SEQ ID NO 1, 2, 9, et des séquences d'une longueur comprise entre 10 et 100 nucléotides, qui s'hybrident préférentiellement à la séquence nucléotidique SEQ ID NO 1, 2, 9 et qui sont capables de se lier à au moins une portion du gène P2, P2A ou P2B ou son ARNm correspondant, et qui sont capables de distinguer les espèces Candida des autres espèces de levure et/ou fongiques, et des séquences d'une longueur comprise entre 10 et 100 nucléotides qui leur sont complémentaires et qui sont capables de distinguer les espèces Candida des autres espèces de levure et/ou fongiques.

5. Procédé permettant de distinguer une espèce de levure ou fongique cible des autres espèces de levure ou fongiques dans un échantillon d'essai, comprenant les étapes consistant à :
(i) mélanger l'échantillon d'essai avec au moins une sonde oligonucléotidique capable de se lier à au moins une portion des gènes P2, P2A et/ou P2B de l'espèce cible ou son ARNm correspondant dans des conditions appropriées ;
(ii) hybrider, dans des conditions de stringence élevée, tout acide nucléique qui peut être présent dans l'échantillon d'essai avec l'oligonucléotide pour former un duplex sonde:cible ; et
(iii) déterminer si un duplex sonde:cible est présent, la présence du duplex identifiant positivement la présence de l'organisme cible dans l'échantillon d'essai, où la sonde est choisie dans le groupe constitué par SEQ ID NO 9, des séquences d'une longueur comprise entre 10 et 100 nucléotides, qui s'hybrident préférentiellement à la séquence nucléotidique SEQ ID NO 9 et sont capables de distinguer les espèces Candida des autres espèces de levure et/ou fongiques, et des séquences d'une longueur comprise entre 10 et 100 nucléotides qui leur sont complémentaires, également capables de servir de sonde pour le gène P2B.

6. Utilisation d'une trousse telle que revendiquée dans l'une quelconque des revendications 1 à 3 ou d'une molécule d'acide nucléique telle que revendiquée dans la revendication 4, dans un essai de diagnostic pour mesurer le titre d'une espèce de levure ou fongique cible chez un patient ; dans un essai de diagnostic pour détecter la présence d'une espèce de levure ou fongique cible ; ou dans un essai de diagnostic pour mesurer une contamination fongique ou par une levure cible dans un environnement.

7. Procédé pour évaluer l'efficacité d'un régime de traitement conçu pour réduire le titre d'une levure et/ou le titre fongique chez un patient, comprenant l'utilisation d'une trousse telle que revendiquée dans l'une quelconque des revendications 1 à 3, ou d'une molécule d'acide nucléique telle que revendiquée dans la revendication 4, à une ou plusieurs étapes clés du régime de traitement, où les étapes clés comprennent la période avant le traitement, durant le traitement et après le traitement.

8. Utilisation telle que revendiquée dans la revendication 6, où l'environnement est un hôpital, un échantillon de nourriture, un échantillon environnemental, par exemple, l'eau, un échantillon industriel tel qu'un échantillon en cours de fabrication ou un produit final nécessitant un contrôle de la charge microbienne ou un contrôle de qualité.
